(19) Europäisches Patentamt
European Patent Office
Office européen des brevets

(11) Veröffentlichungsnummer: **0 300 307**
**A2**

(12) **EUROPÄISCHE PATENTANMELDUNG**

(21) Anmeldenummer: **88111026.6**

(22) Anmeldetag: **11.07.88**

(51) Int. Cl.⁴: **C07D 405/04 , C07D 325/00 , A01N 43/24 , A01N 43/38 , //A01N37/32**

(30) Priorität: **21.07.87 DE 3724098**

(43) Veröffentlichungstag der Anmeldung: **25.01.89 Patentblatt 89/04**

(84) Benannte Vertragsstaaten: **BE CH DE FR GB IT LI NL**

(71) Anmelder: **BAYER AG**
**D-5090 Leverkusen 1, Bayerwerk(DE)**

(72) Erfinder: **Daum, Werner, Dr.**
**Bärenstrasse 18**
**D-4150 Krefeld(DE)**
Erfinder: **Fengler, Gerd, Dr.**
**Bethelstrasse 16**
**D-4150 Krefeld(DE)**
Erfinder: **Fischer, Reiner, Dr.**
**Rembrandtstrasse 15**
**D-4019 Monheim(DE)**
Erfinder: **Heywang, Gerhard, Dr.**
**Nittumer Weg 4**
**D-5060 Bergisch Gladbach 2(DE)**
Erfinder: **Marhold, Albrecht, Dr.**
**Carl-Duisberg-Strasse 329**
**D-5090 Leverkusen(DE)**
Erfinder: **Schallner, Otto, Dr.**
**Noldeweg 22**
**D-4019 Monheim(DE)**
Erfinder: **Santel, Hans-Joachim, Dr.**
**Grünstrasse 9a**
**D-5090 Leverkusen(DE)**
Erfinder: **Schmidt, Robert R., Dr.**
**Im Waldwinkel 110**
**D-5060 Bergisch Gladbach 2(DE)**
Erfinder: **Lürssen, Klaus, Dr.**
**August-Kierspel-Strasse 145**
**D-5060 Bergisch Gladbach 2(DE)**
Erfinder: **Strang, Harry, Dr.**
**Unterdorfstrasse 6a**
**D-4000 Düsseldorf 31(DE)**

(54) **N-Aryl-Stickstoffheterocyclen.**

(57) Die Erfindung betrifft N-Aryl-Stickstoffheterocyclen der allgemeinen Formel (I),

**EP 0 300 307 A2**

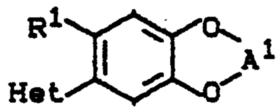

(I)

in welcher
Het für einen Heterocyclus der Formel

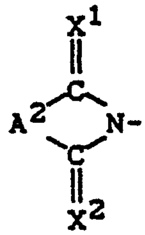

oder

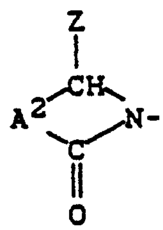

steht

und die übrigen Substituenten die in der Beschreibung angegebene Bedeutung haben, mehrere Verfahren sowie neue Zwischenprodukte zu deren Herstellung und ihre Verwendung als Herbizide und Pflanzenwuchsregulatoren.

## N-Aryl-Stickstoffheterocyclen

Die Erfindung betrifft neue N-Aryl-Stickstoffheterocyclen, mehrere Verfahren zu ihrer Herstellung und ihre Verwendung als Herbizide und Pflanzenwachstumsregulatoren.

Es ist bereits bekannt, daß bestimmte N-Aryl-Stickstoffheterocyclen, wie beispielsweise das N-(2-Fluor-4-chlor-5-propargyloxy-phenyl)-$\Delta^1$-tetrahydrophthalimid oder das 2-(4-(Chlor-2-fluor-5-methoxycarbonylmethoxyphenyl)-4,5,6,7-tetrahydro-2H-isoindol-1,3-dion herbizide Eigenschaften besitzen (vgl. z.B. EP 61 741, EP 83 055 und DE-OS 3 013 162).

Die herbizide Wirksamkeit dieser vorbekannten Verbindungen gegenüber Problemunkräutern ist jedoch ebenso wie ihre Verträglichkeit gegenüber bestimmten Kulturpflanzen nicht in allen Anwendungsgebieten völlig zufriedenstellend.

Über eine pflanzenwachstumsregulierende Wirkung der vorbekannten Verbindungen ist bisher nichts bekannt.

Es wurden neue N-Aryl-Stickstoffheterocyclen der allgemeinen Formel (I),

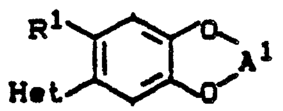

(I)

in welcher

Het für einen Heterocyclus der Formel

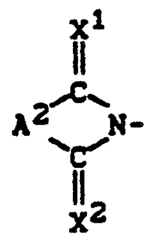

oder

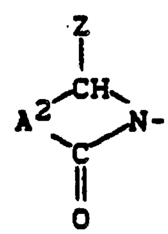

steht,

$R^1$ für Wasserstoff oder Halogen steht,

$A^1$ für einen Rest der Formel

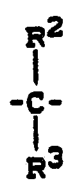

;

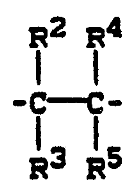

oder

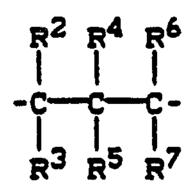

steht,

wobei

$A^2$ für einen Rest der Formel

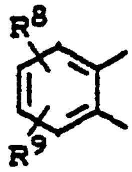 ; 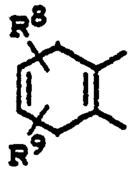 ; 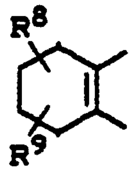 ; 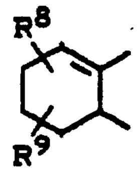 ;

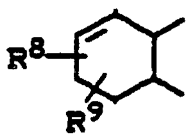 ; 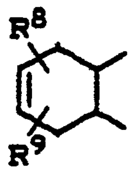 oder 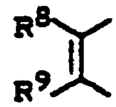 steht,

$X^1$ und $X^2$ jeweils für Sauerstoff oder Schwefel stehen,
Z für Wasserstoff, Hydroxy oder Chlor steht,
$R^2$, $R^3$, $R^4$, $R^5$, $R^6$ und $R^7$ unabhängig voneinander jeweils für Wasserstoff, Halogen, Alkyl oder Halogenalkyl stehen und
$R^8$ und $R^9$ unabhängig voneinander jeweils für Wasserstoff, Halogen, Alkyl oder Halogenalkyl stehen, gefunden.

Weiterhin wurde gefunden, daß man die neuen N-Aryl-Stickstoffheterocyclen der allgemeinen Formel (I),

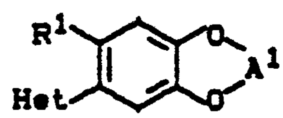 (I)

in welcher
Het für einen Heterocyclus der Formel

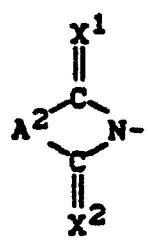 oder 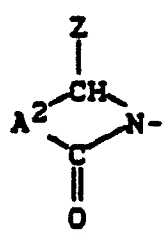 steht,

$R^1$ für Wasserstoff oder Halogen steht,
$A^1$ für einen Rest der Formel

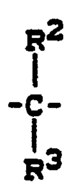 ; 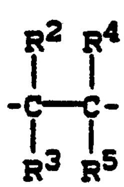 oder 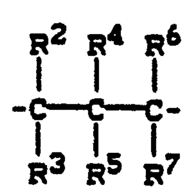 steht,

wobei
$A^2$ für einen Rest der Formel

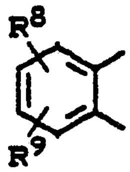 ; 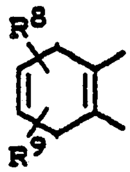 ; 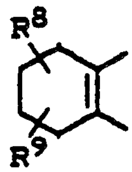 ; 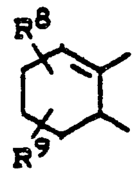 ;

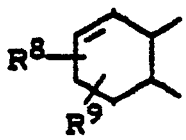 ; 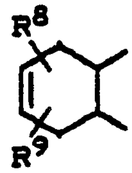 oder 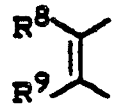 steht,

$X^1$ und $X^2$ jeweils für Sauerstoff oder Schwefel stehen,
Z für Wasserstoff, Hydroxy oder Chlor steht,
$R^2$, $R^3$, $R^4$, $R^5$, $R^6$ und $R^7$ unabhängig voneinander jeweils für Wasserstoff, Halogen, Alkyl oder Halogenalkyl stehen und
$R^8$ und $R^9$ unabhängig voneinander jeweils für Wasserstoff, Halogen, Alkyl oder Halogenalkyl stehen,
nach einem der im Folgenden beschriebenen Verfahren erhält:

(a) Man erhält N-Arylstickstoffheterocyclen der Formel (Ia),

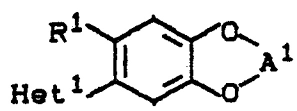 (Ia)

in welcher
$Het^1$ für einen Rest

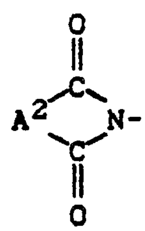

steht und
$A^1$, $A^2$ und $R^1$ die oben angegebene Bedeutung haben,
wenn man Anhydride der Formel (II),

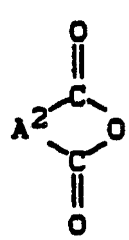 (II)

in welcher
$R^2$ die oben angegebene Bedeutung hat,
mit Aminen der Formel (III),

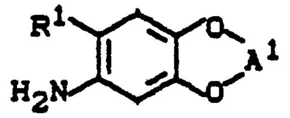

(III)

in welcher
$R^1$ und $A^1$ die oben angegebene Bedeutung haben,
gegebenenfalls in Gegenwart eines Verdünnungsmittels und gegebenenfalls in Gegenwart eines Reaktionshilfsmittels umsetzt;

(b) man erhält N-Aryl-Stickstoffheterocyclen der Formel (Ib),

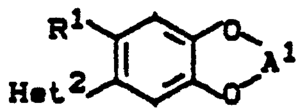

(Ib)

in welcher
$Het^2$ für einen Rest

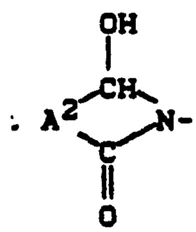

steht und
$A^1$, $A^2$ und $R^1$ die oben angegebene Bedeutung haben,
wenn man N-Aryl-Stickstoffheterocyclen der Formel (Ia),

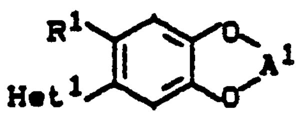

(Ia)

in welcher
$Het^1$ für einen Rest

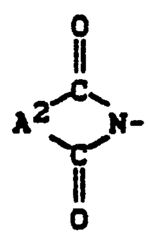

steht und
$A^1$, $A^2$ und $R^1$ die oben angegebene Bedeutung haben,
mit einem Reduktionsmittel, gegebenenfalls in Gegenwart eines Verdünnungsmittels umsetzt;

(c) man erhält N-Aryl-Stickstoffheterocyclen der Formel (Ic),

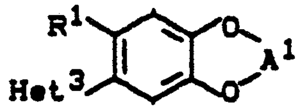

(Ic)

in welcher
Het³ für einen Rest

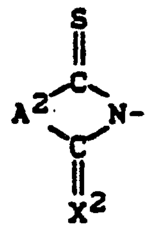

steht und
$A^1$, $A^2$, $R^1$ und $X^2$ die oben angegebene Bedeutung haben,
wenn man N-Aryl-Stickstoffheterocyclen der Formel (Ia),

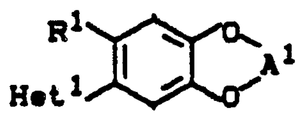

(Ia)

in welcher
$Het^1$ für einen Rest

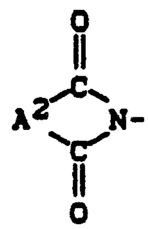

steht und
$A^1$, $A^2$ und $R^1$ die oben angegebene Bedeutung haben,
mit "Lawessons Reagenz" der Formel (IV),

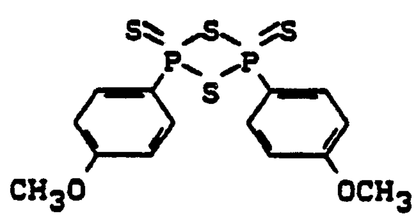

(IV)

gegebenenfalls in Gegenwart eines Verdünnungsmittels umsetzt;

(d) man erhält N-Aryl-Stickstoffheterocyclen der Formel (Id),

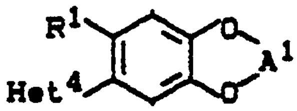

(Id)

in welcher
Het⁴ für einen Rest

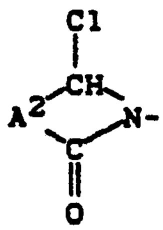

steht und
$A^1$, $A^2$ und $R^1$ die oben angegebene Bedeutung haben,
wenn man N-Aryl-Stickstoffheterocyclen der Formel (Ib),

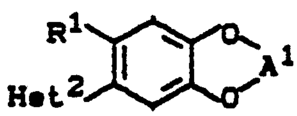

(Ib)

in welcher
$Het^2$ für einen Rest

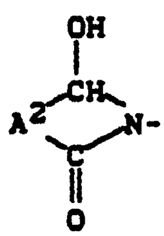

steht und
$A^1$, $A^2$ und $R^1$ die oben angegebene Bedeutung haben,
mit Thionylchlorid gegebenenfalls in Gegenwart eines Verdünnungsmittels und gegebenenfalls in Gegenwart eines Reaktionshilfsmittels umsetzt;

(e) man erhält N-Aryl-Stickstoffheterocyclen der Formel (Ie),

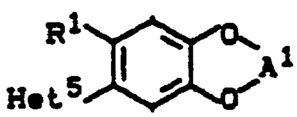

(Ie)

in welcher
$Het^5$ für einen Rest

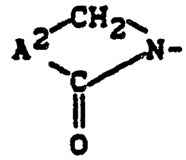

steht und

$A^1$, $A^2$ und $R^1$ die oben angegebene Bedeutung haben,

wenn man N-Aryl-Stickstoffheterocyclen der Formel (Id),

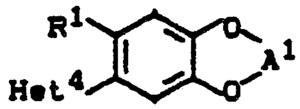 (Id)

in welcher

$Het^4$ für einem Rest

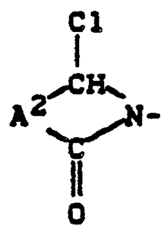

steht und

$A^1$, $A^2$ und $R^1$ die oben angegebene Bedeutung haben,

mit molekularem Wasserstoff in Gegenwart eines Katalysators sowie gegebenenfalls in Gegenwart eines Verdünnungsmittels und gegebenenfalls in Gegenwart eines Säurebindemittels umsetzt.

Schließlich wurde gefunden, daß die neuen N-Aryl-Stickstoffheterocyclen der allgemeinen Formel (I) herbizide und pflanzenwachstumsregulierende Eigenschaften besitzen.

Überraschenderweise zeigen die erfindungsgemäßen N-Aryl-Stickstoffheterocyclen der allgemeinen Formel (I) neben einer erheblich besseren herbiziden Wirksamkeit gegenüber wichtigen Problemunkräutern gleichzeitig eine deutlich verbesserte Verträglichkeit gegenüber wichtigen Kulturpflanzen im Vergleich zu den aus dem Stand der Technik bekannten N-Aryl-Stickstoffheterocyclen, wie beispielsweise das N-(2-Fluor-4-chlor-5-propargyloxyphenyl)-$\Delta^1$-tetrahydrophthalimid oder das 2-(4-Chlor-2-fluor-5-methoxycarbonylmethoxyphenyl)-4,5,6,7-tetrahydro-2H-isoindol-1,3-dion, welche chemisch und wirkungsmäßig naheliegende Verbindungen sind. Darüberhinaus zeigen die erfindungsgemäßen Verbindungen der Formel (I) unerwarteterweise zusätzlich eine pflanzenwachstumsregulierende Wirkung.

Die erfindungsgemäßen N-Aryl-Stickstoffheterocyclen sind durch die Formel (I) allgemein definiert.

Bevorzugt sind Verbindungen der Formel (I), bei welchen

Het für einen Heterocyclus der Formel

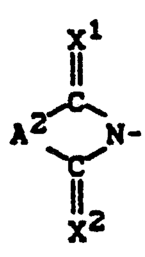 oder 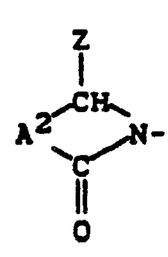 steht,

$R^1$ für Wasserstoff, Fluor, Chlor oder Brom steht und

$A^1$ für einen Rest der Formel

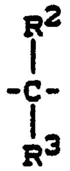 ; 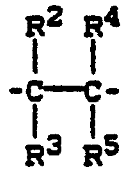 oder 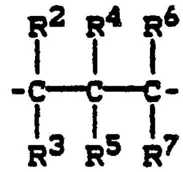 steht,

wobei

$A^2$ für einen Rest der Formel

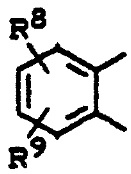 ; 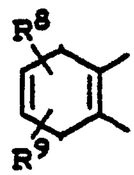 ; 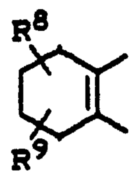 ; 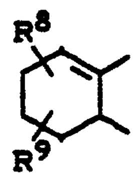 ;

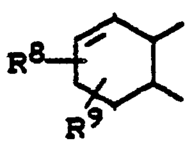 ; 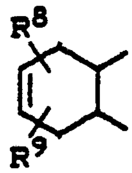 oder 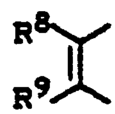 steht,

$X^1$ und $X^2$ für Sauerstoff oder Schwefel stehen,
Z für Wasserstoff, Hydroxy oder Chlor steht,
$R^2$, $R^3$, $R^4$, $R^5$, $R^6$ und $R^7$ unabhängig voneinander jeweils für Wasserstoff, Fluor, Chlor, Brom oder für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen - insbesondere Fluor, Chlor oder Brom -stehen und
$R^8$ und $R^9$ unabhängig voneinander jeweils für Wasserstoff, Fluor, Chlor, Brom oder für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen, insbesondere Fluor, Chlor oder Brom stehen.

Besonders bevorzugt sind Verbindungen der Formel (I), bei welchen
Het für einen Heterocyclus der Formel

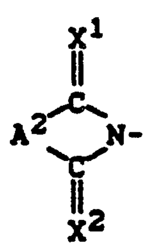 oder 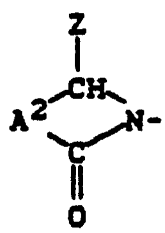 steht,

$R^1$ für Wasserstoff, Fluor oder Chlor steht und
$A^1$ für einen Rest der Formel

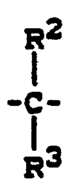 oder 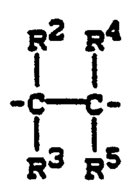 steht, wobei

$A^2$ für einen der Reste

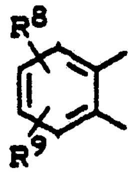 ; 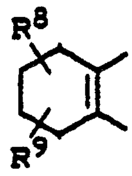 oder 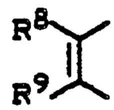 steht,

$X^1$ und $X^2$ für Sauerstoff oder Schwefel stehen,
Z für Wasserstoff, Hydroxy oder Chlor steht,
$R^2$, $R^3$, $R^4$ und $R^5$ unabhängig voneinander für Wasserstoff, Fluor, Chlor, Methyl oder Trifluormethyl stehen und
$R^8$ und $R^9$ unabhängig voneinander für Wasserstoff, Chlor, Methyl oder Trifluormethyl stehen.

Ganz besonders bevorzugt sind Verbindungen der Formel (I), bei welchen
Het für einen Heterocyclus der Formel

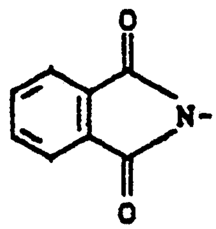 ; 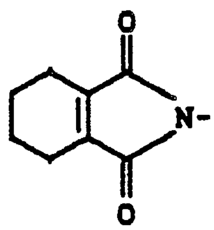 oder 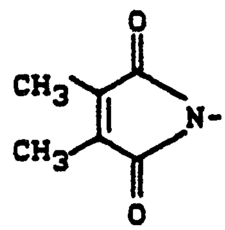

steht,
$R^1$ für Wasserstoff oder Fluor steht und
$A^1$ für einen Rest der Formel

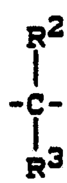 oder 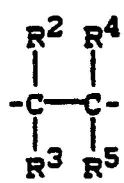 steht, wobei

$R^2$, $R^3$, $R^4$ und $R^5$ unabhängig voneinander für Wasserstoff, Fluor, Chlor oder Methyl stehen.

Im einzelnen seien außer den bei den Herstellungsbeispielen genannten Verbindungen die folgenden N-Aryl-Stickstoffheterocyclen der allgemeinen Formel (I) genannt:

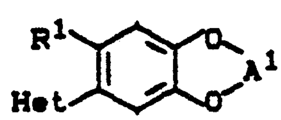 (I)

| Het | $R^1$ | $A^1$ |
|---|---|---|
| 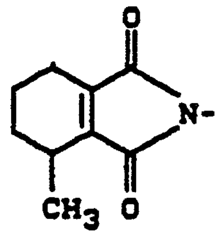 | F | $-CF_2-$ |
| 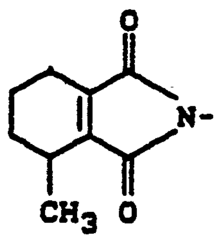 | H | $-CF_2-$ |
| 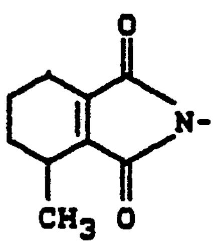 | F | $-CH_2-$ |
| 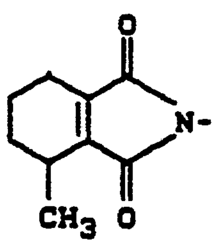 | F | $-CH_2-CH_2-$ |
| 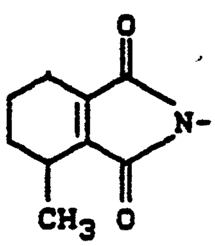 | F | $-CF_2-CF_2-$ |

| Het | $R^1$ | $A^1$ |
|---|---|---|
| 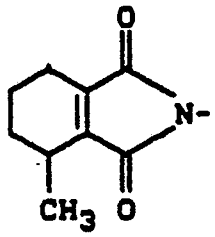 | F | $-CF_2-CHF-$ |
| 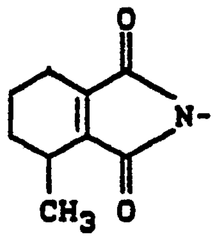 | F | $-CF_2-CClF-$ |
| 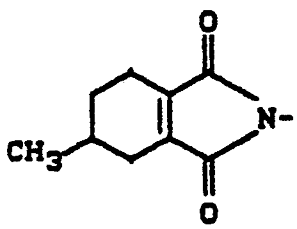 | F | $-CF_2-$ |
| 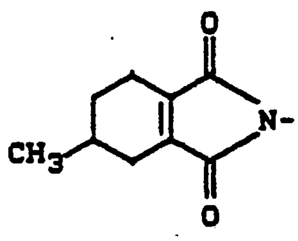 | H | $-CF_2-$ |
| 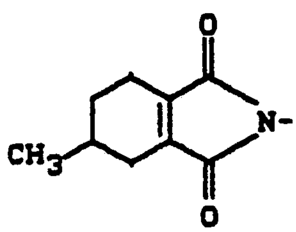 | F | $-CH_2-$ |

| Het | $R^1$ | $A^1$ |
|---|---|---|
| 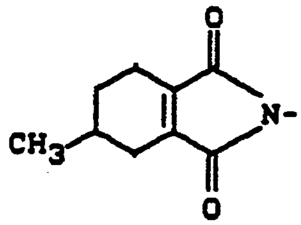 | F | $-CH_2-CH_2-$ |
| 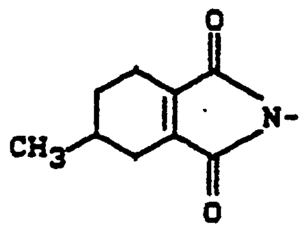 | F | $-CF_2-CF_2-$ |
| 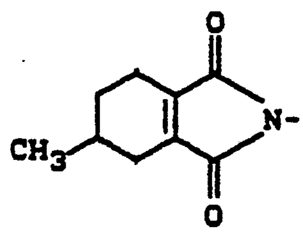 | F | $-CF_2-CHF-$ |
| 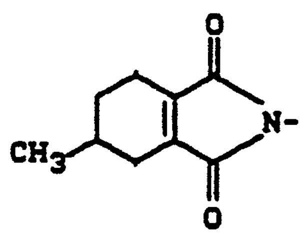 | F | $-CF_2-CClF-$ |
| 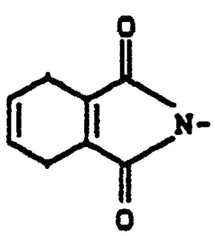 | F | $-CF_2-$ |

| Het | $R^1$ | $A^1$ |
|---|---|---|
| 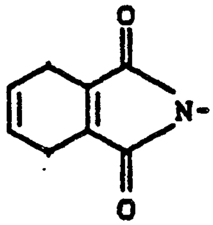 | H | $-CF_2-$ |
| 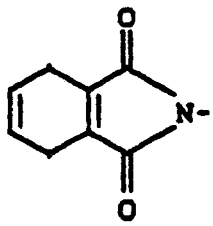 | F | $-CH_2-$ |
| 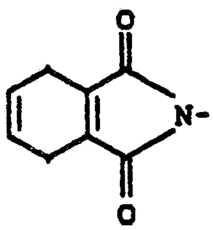 | F | $-CH_2-CH_2-$ |
| 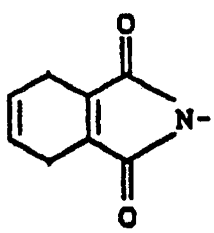 | F | $-CF_2-CF_2-$ |
| 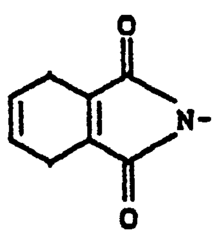 | F | $-CF_2-CFCl-$ |

| Het | $R^1$ | $A^1$ |
|---|---|---|
| 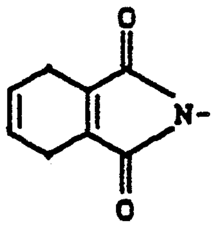 | F | $-CF_2-CHF-$ |
| 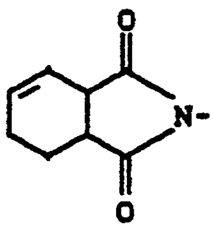 | F | $-CF_2-$ |
| 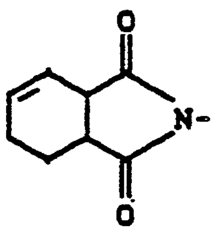 | H | $-CF_2-$ |
| 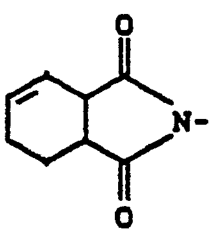 | F | $-CH_2-$ |
| 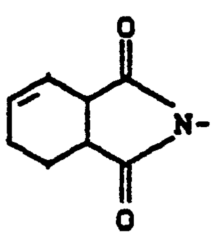 | F | $-CH_2-CH_2-$ |

| Het | $R^1$ | $A^1$ |
|---|---|---|
| 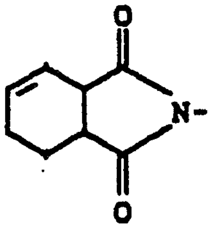 | F | $-CF_2-CF_2-$ |
| 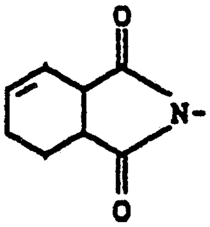 | F | $-CF_2-CHF-$ |
| 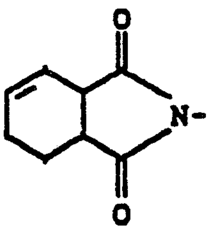 | F | $-CF_2-CClF-$ |
| 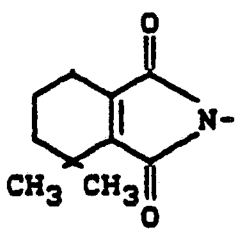 | F | $-CF_2-$ |
| 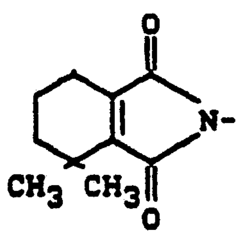 | H | $-CF_2-$ |

| Het | $R^1$ | $A^1$ |
|---|---|---|
| 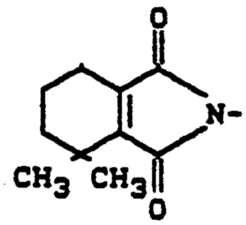 | F | $-CH_2-$ |
| 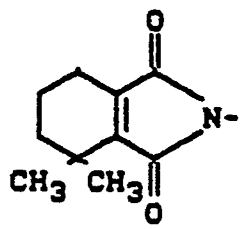 | F | $-CH_2-CH_2-$ |
| 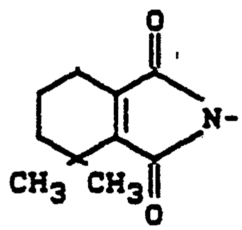 | F | $-CF_2-CF_2-$ |
| 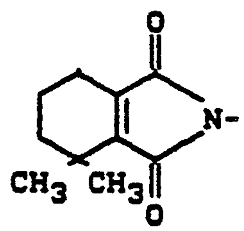 | F | $-CF_2-CFCl-$ |
| 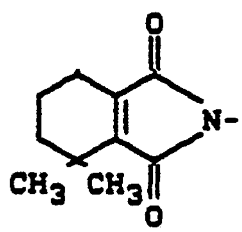 | F | $-CF_2-CHF-$ |

| Het | $R^1$ | $A^1$ |
|---|---|---|
| 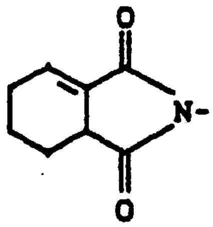 | F | $-CF_2-$ |
| 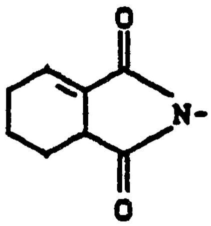 | H | $-CF_2-$ |
| 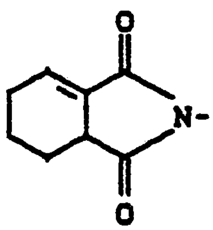 | F | $-CH_2-$ |
| 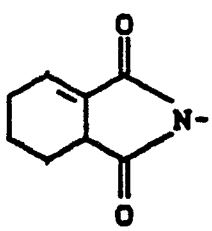 | F | $-CH_2-CH_2-$ |
| 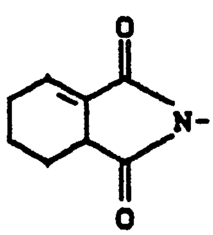 | F | $-CF_2-CF_2-$ |

| Het | $R^1$ | $A^1$ |
|---|---|---|
| 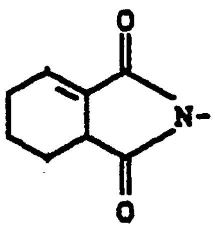 | F | $-CF_2-CHF-$ |
| 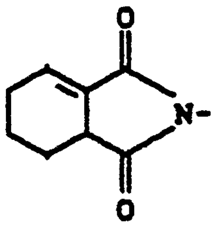 | F | $-CF_2-CClF-$ |
| 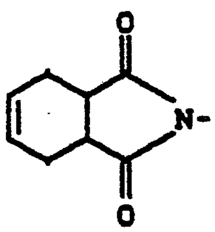 | F | $-CF_2-$ |
| 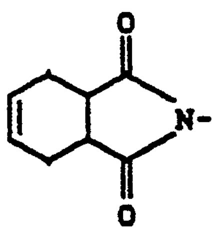 | H | $-CF_2-$ |
| 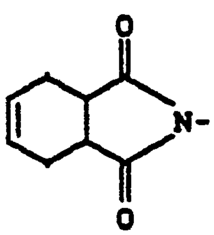 | F | $-CH_2-$ |

| Het | $R^1$ | $A^1$ |
|---|---|---|
| 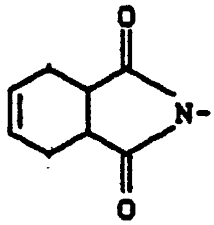 | F | $-CH_2-CH_2-$ |
| 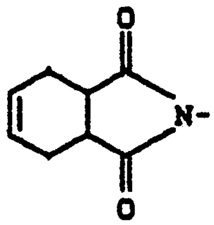 | F | $-CF_2-CF_2-$ |
| 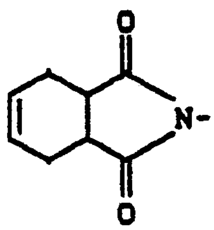 | F | $-CF_2-CFCl-$ |
| 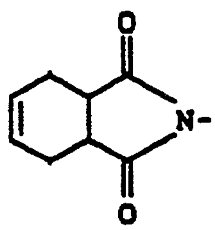 | F | $-CF_2-CHF-$ |
| 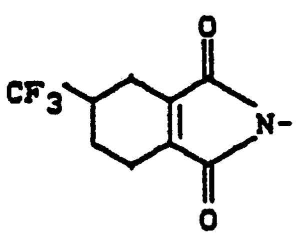 | F | $-CF_2-$ |

| Het | $R^1$ | $A^1$ |
|---|---|---|
| 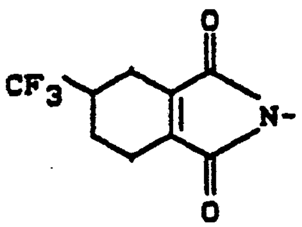 | H | $-CF_2-$ |
| 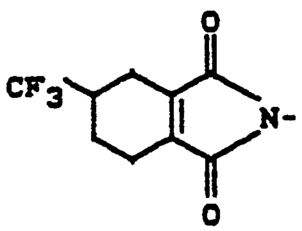 | F | $-CH_2-$ |
| 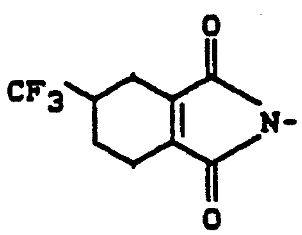 | F | $-CH_2-CH_2-$ |
| 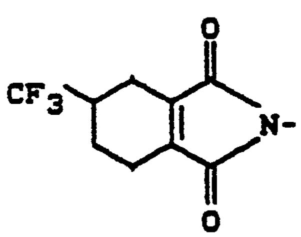 | F | $-CF_2-CF_2-$ |
| 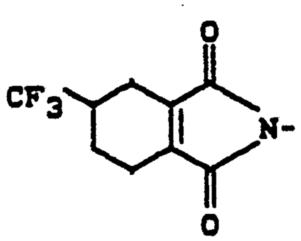 | F | $-CF_2-CHF-$ |

| Het | $R^1$ | $A^1$ |
|---|---|---|
| 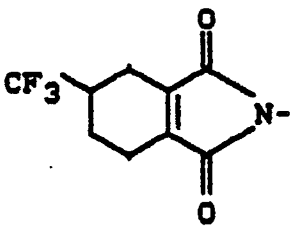 | F | $-CF_2-CClF-$ |
| 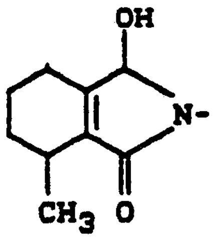 | F | $-CF_2-$ |
| 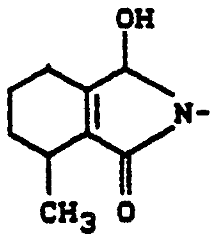 | H | $-CF_2-$ |
| 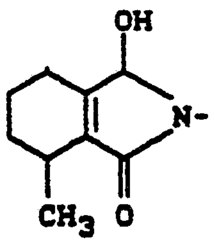 | F | $-CH_2-$ |
| 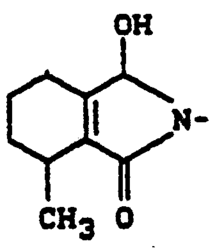 | F | $-CH_2-CH_2-$ |

| Het | $R^1$ | $A^1$ |
|---|---|---|
| 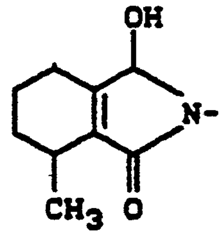 | F | $-CF_2-CF_2-$ |
| 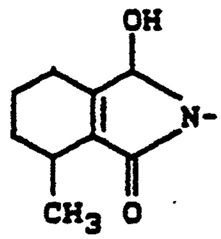 | F | $-CF_2-CFCl-$ |
| 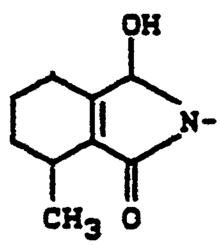 | F | $-CF_2-CHF-$ |
| 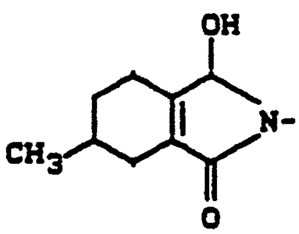 | F | $-CF_2-$ |
| 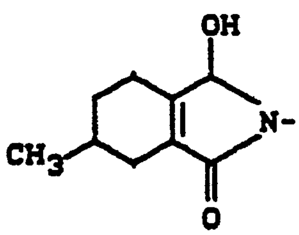 | H | $-CF_2-$ |

| Het | $R^1$ | $A^1$ |
|---|---|---|
| 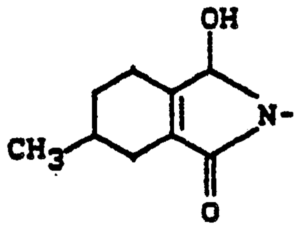 | F | $-CH_2-$ |
| 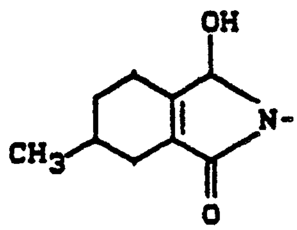 | F | $-CH_2-CH_2-$ |
| 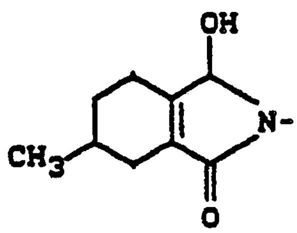 | F | $-CF_2-CF_2-$ |
| 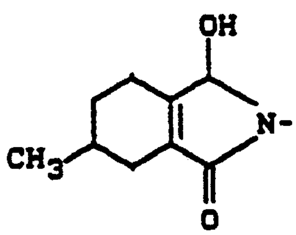 | F | $-CF_2-CHF-$ |
| 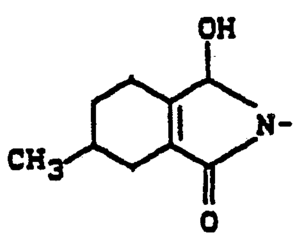 | F | $-CF_2-CClF-$ |

| Het | $R^1$ | $A^1$ |
|---|---|---|
| 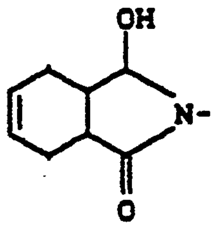 | F | $-CF_2-$ |
| 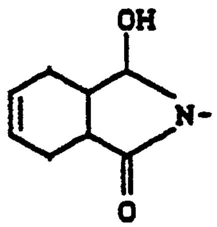 | H | $-CF_2-$ |
| 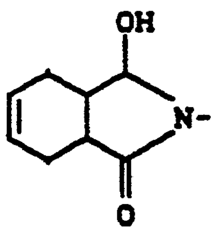 | F | $-CH_2-$ |
| 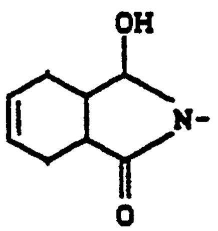 | F | $-CH_2-CH_2-$ |
| 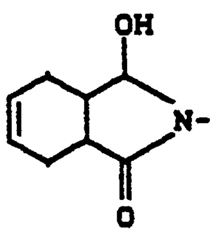 | F | $-CF_2-CF_2-$ |

| Het | $R^1$ | $A^1$ |
|---|---|---|
| 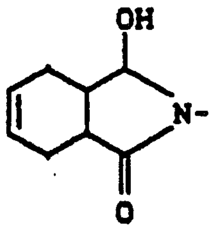 | F | $-CF_2-CFCl-$ |
| 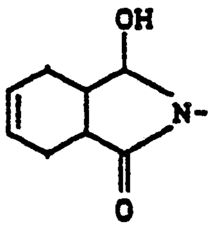 | F | $-CF_2-CHF-$ |
| 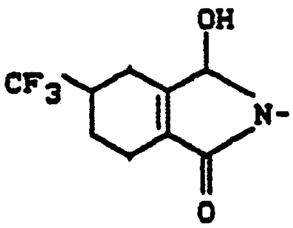 | F | $-CF_2-$ |
| 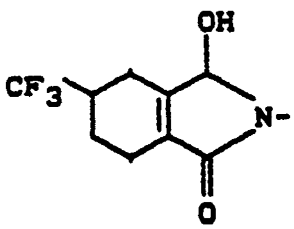 | H | $-CF_2-$ |
| 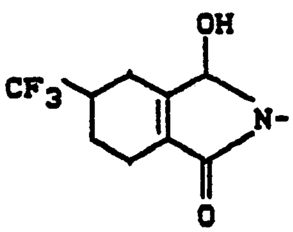 | F | $-CH_2-$ |

| Het | $R^1$ | $A^1$ |
|---|---|---|
| 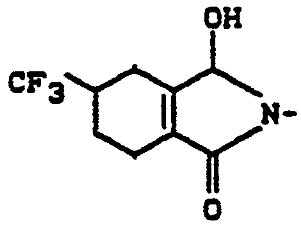 | F | $-CH_2-CH_2-$ |
| 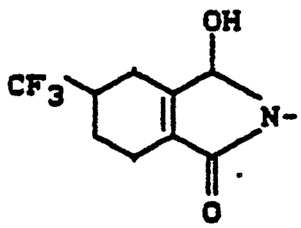 | F | $-CF_2-CF_2-$ |
| 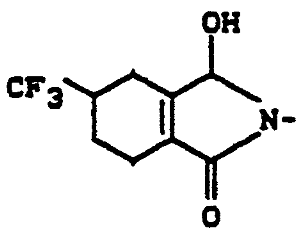 | F | $-CF_2-CHF-$ |
| 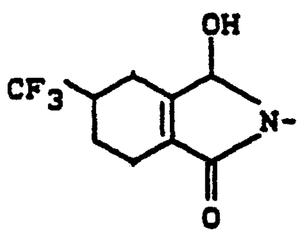 | F | $-CF_2-CClF-$ |
| 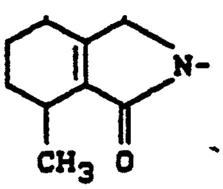 | F | $-CF_2-$ |

| Het | $R^1$ | $A^1$ |
|---|---|---|
| 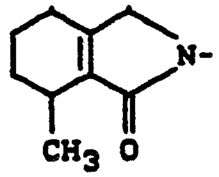 | H | $-CF_2-$ |
| 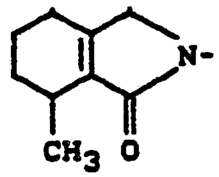 | F | $-CH_2-$ |
| 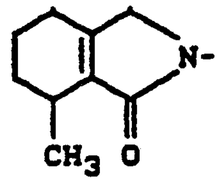 | F | $-CH_2-CH_2-$ |
| 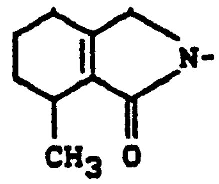 | F | $-CF_2-CF_2-$ |
| 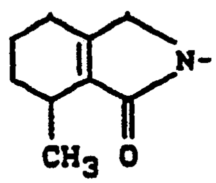 | F | $-CF_2-CFCl-$ |
| 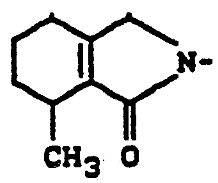 | F | $-CF_2-CHF-$ |

| Het | $R^1$ | $A^1$ |
|---|---|---|
| 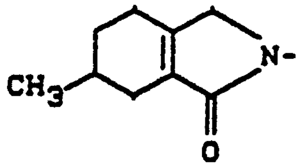 | F | $-CF_2-$ |
| 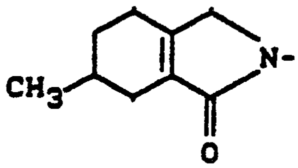 | H | $-CF_2-$ |
| 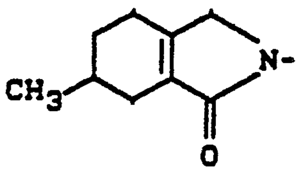 | F | $-CH_2-$ |
| 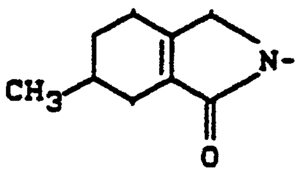 | F | $-CH_2-CH_2-$ |
| 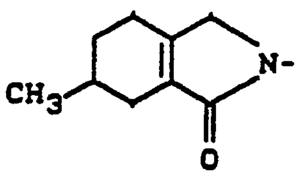 | F | $-CF_2-CF_2-$ |
| 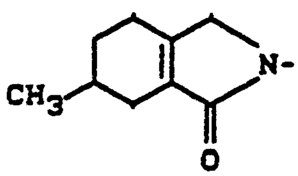 | F | $-CF_2-CHF-$ |

| Het | $R^1$ | $A^1$ |
|---|---|---|
| 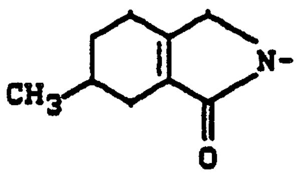 | F | $-CF_2-CClF-$ |
| 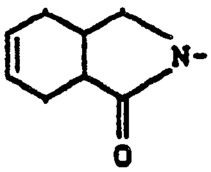 | F | $-CF_2-$ |
| 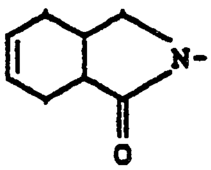 | H | $-CF_2-$ |
| 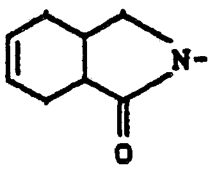 | F | $-CH_2-$ |
| 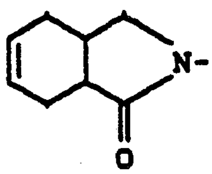 | F | $-CH_2-CH_2-$ |
| 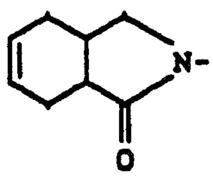 | F | $-CF_2-CF_2-$ |

| Het | $R^1$ | $A^1$ |
|---|---|---|
| 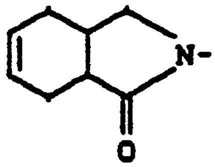 | F | $-CF_2-CFCl-$ |
| 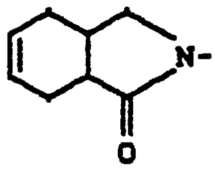 | F | $-CF_2-CHF-$ |
| 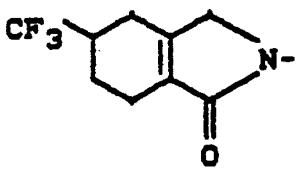 | F | $-CF_2-$ |
| 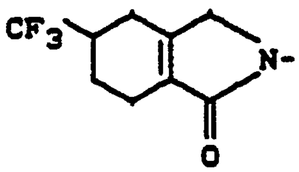 | H | $-CF_2-$ |
| 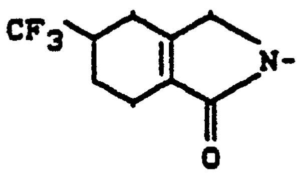 | F | $-CH_2-$ |
| 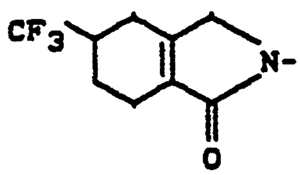 | F | $-CH_2-CH_2-$ |

| Het | $R^1$ | $A^1$ |
|---|---|---|
| 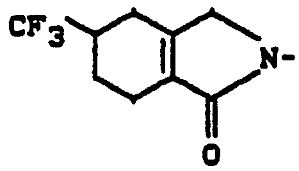 | F | $-CF_2-CF_2-$ |
| 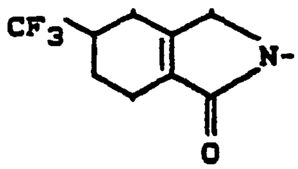 | F | $-CF_2-CHF-$ |
| 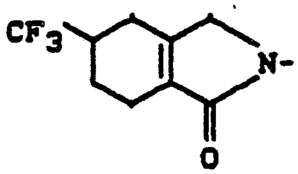 | F | $-CF_2-CClF-$ |
| 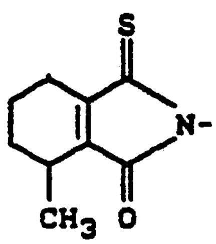 | F | $-CF_2-$ |
| 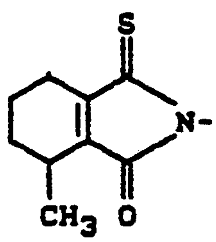 | H | $-CF_2-$ |

| Het | $R^1$ | $A^1$ |
| --- | --- | --- |
| 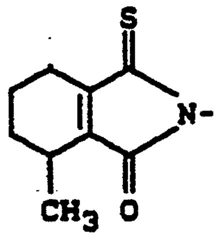 | F | $-CH_2-$ |
| 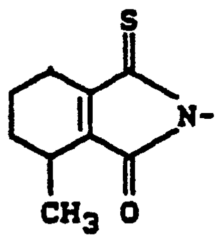 | F | $-CH_2-CH_2-$ |
| 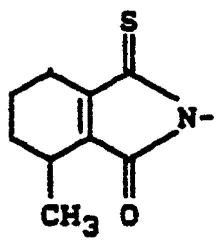 | F | $-CF_2-CF_2-$ |
| 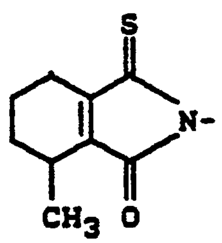 | F | $-CF_2-CFCl-$ |
| 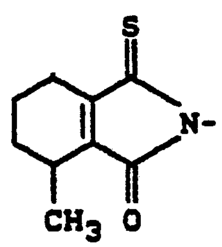 | F | $-CF_2-CHF-$ |

| Het | $R^1$ | $A^1$ |
| --- | --- | --- |
| 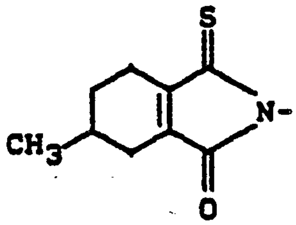 | F | $-CF_2-$ |
| 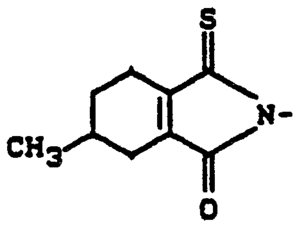 | H | $-CF_2-$ |
| 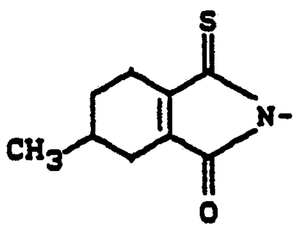 | F | $-CH_2-$ |
| 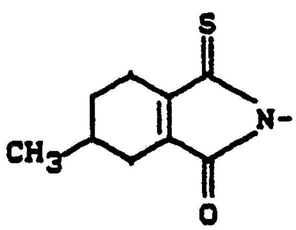 | F | $-CH_2-CH_2-$ |
| 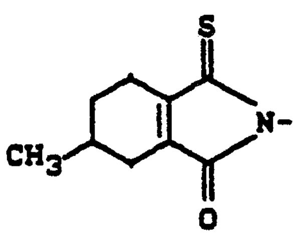 | F | $-CF_2-CF_2-$ |

| Het | $R^1$ | $A^1$ |
|---|---|---|
| 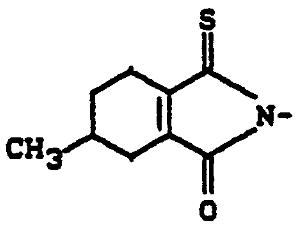 | F | $-CF_2-CHF-$ |
| 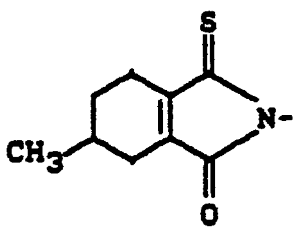 | F | $-CF_2-CClF-$ |
| 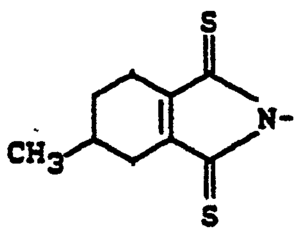 | F | $-CF_2-$ |
| 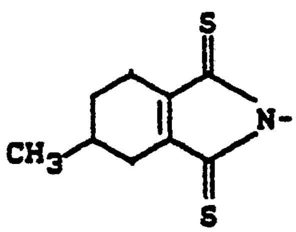 | H | $-CF_2-$ |
| 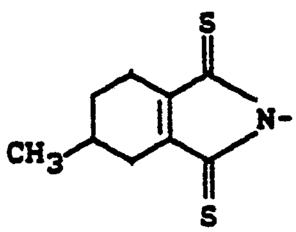 | F | $-CH_2-$ |

| Het | $R^1$ | $A^1$ |
| --- | --- | --- |
| 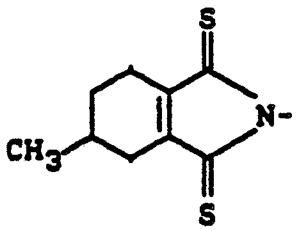 | F | $-CH_2-CH_2-$ |
| 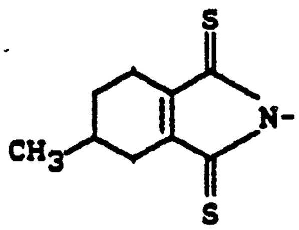 | F | $-CF_2-CF_2-$ |
| 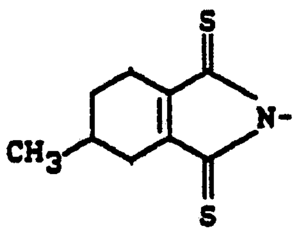 | F | $-CF_2-CFCl-$ |
| 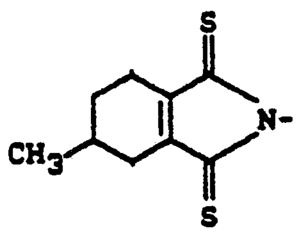 | F | $-CF_2-CHF-$ |
| 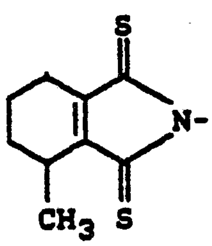 | F | $-CF_2-$ |

| Het | $R^1$ | $A^1$ |
|---|---|---|
| 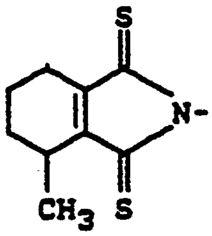 | H | $-CF_2-$ |
| 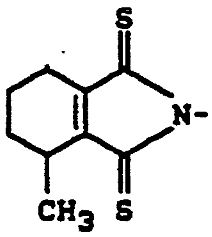 | F | $-CH_2-$ |
| 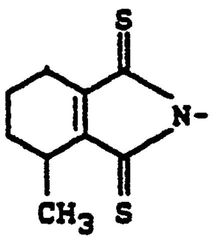 | F | $-CH_2-CH_2-$ |
| 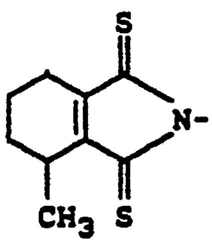 | F | $-CF_2-CF_2-$ |
| 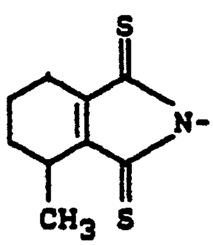 | F | $-CF_2-CHF-$ |

| Het | $R^1$ | $A^1$ |
|---|---|---|
| 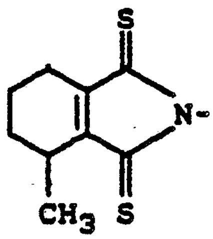 | F | $-CF_2-CClF-$ |
| 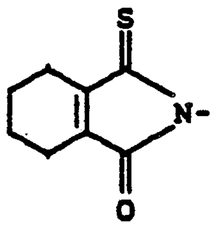 | F | $-CF_2-$ |
| 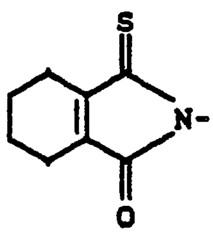 | H | $-CF_2-$ |
| 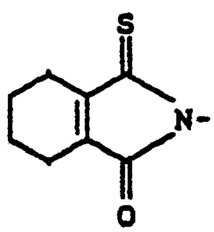 | F | $-CH_2-$ |
| 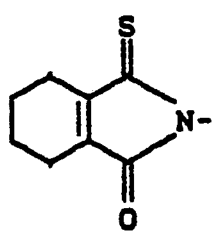 | F | $-CH_2-CH_2-$ |

| Het | $R^1$ | $A^1$ |
|---|---|---|
| 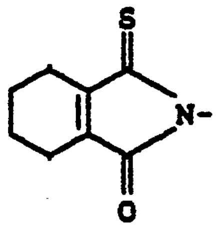 | F | $-CF_2-CF_2-$ |
| 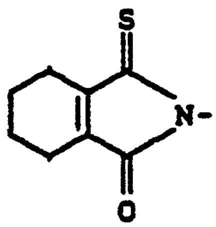 | F | $-CF_2-CFCl-$ |
| 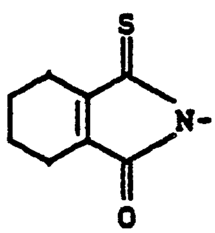 | F | $-CF_2-CHF-$ |

Verwendet man beispielsweise 3,4,5,6-Tetrahydrophthalsäureanhydrid und 2-Fluor-4,5-dioxydifluormethylenanilin als Ausgangsstoffe, so läßt sich der Reaktionsablauf des erfindungsgemäßen Verfahrens (a) durch das folgende Formelschema darstellen:

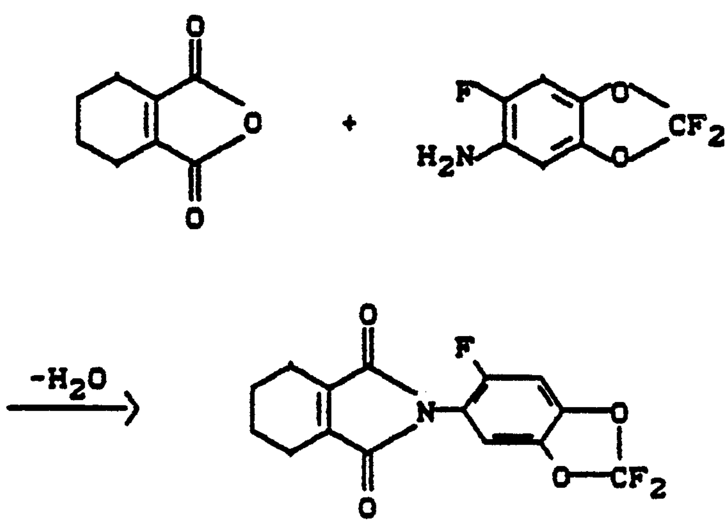


Verwendet man beispielsweise N-(2-Fluor-4,5-difluormethylen-phenyl)-3,4,5,6-tetrahydrophthalimid als Ausgangsverbindung und Natriumborhydrid als Reduktionsmittel, so läßt sich der Reaktionsablauf des erfindungsgemäßen Verfahrens (b) durch das folgende Formelschema darstellen:

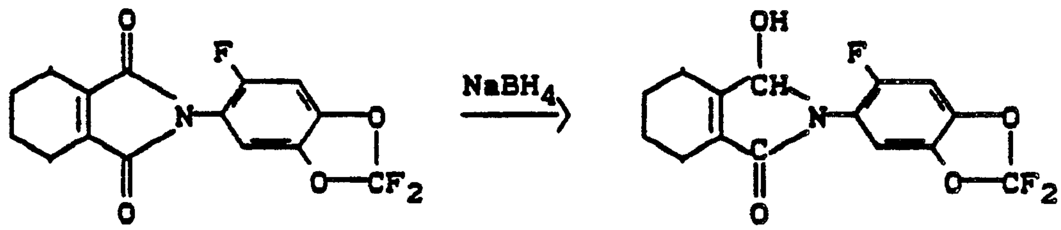


Verwendet man beispielsweise N-(2-Fluor-4,5-dioxytetrafluorethylen-phenyl)-3,4,5,6-tetrahydrophthalimid und "Lawessons Reagenz" als Ausgangsstoffe, so läßt sich der Reaktionsablauf des erfindungsgemäßen Verfahrens (c) durch das folgende Formelschema darstellen:

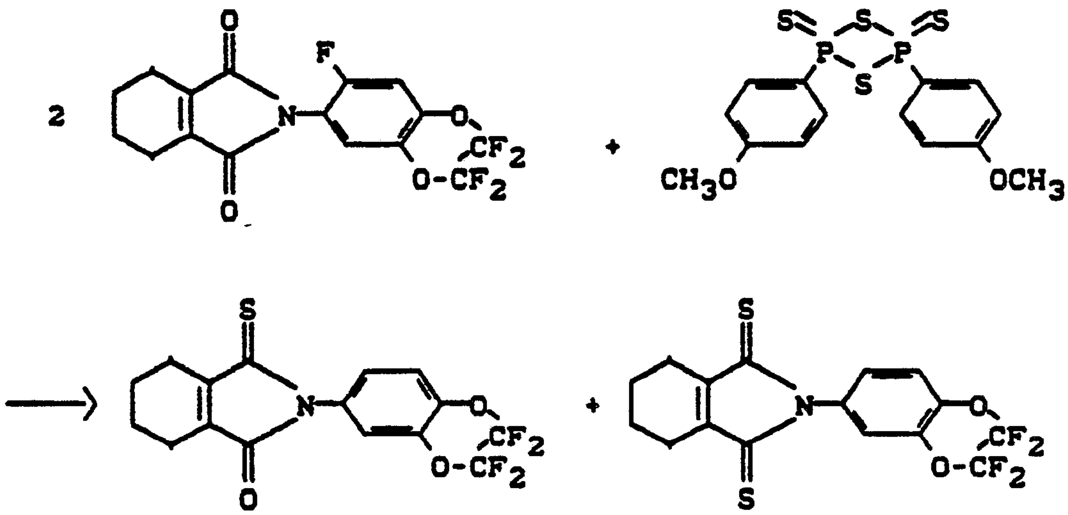

Verwendet man beispielsweise N-(2-Fluor-4,5-dioxydifluormethylen-phenyl)-3,4-dimethyl-Δ$^3$-pyrrolin-5-ol-2-on und Thionylchlorid als Ausgangsstoffe, so läßt sich der Reaktionsablauf des erfindungsgemäßen Verfahrens (d) durch das folgende Formelschema darstellen:

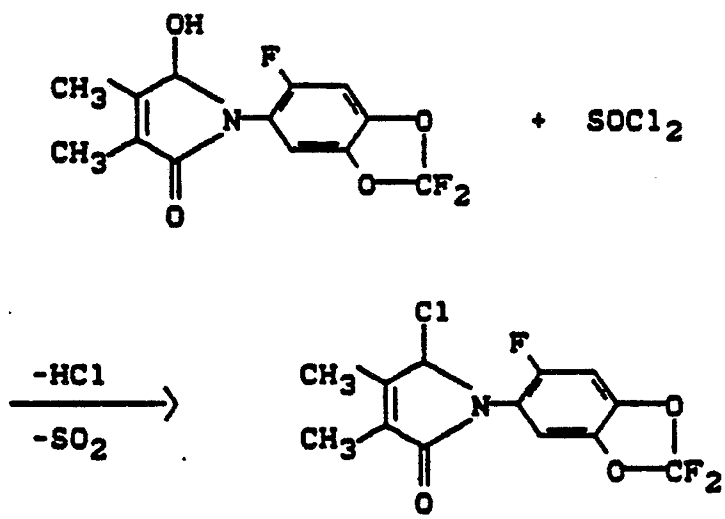


Verwendet man beispielsweise N-(3-Fluor-4,5-dioxymethylen-phenyl)-3,4-dimethyl-5-chlor-Δ$^3$-pyrrolin-2-on als Ausgangsverbindung, so läßt sich der Reaktionsablauf des erfindungsgemäßen Verfahrens (e) durch das folgende Formelschema darstellen:

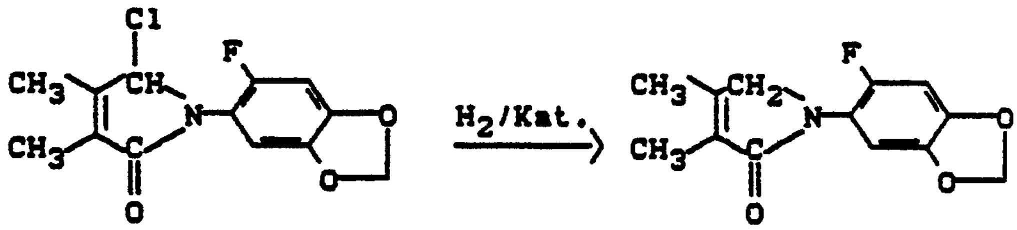


Die zur Durchführung des erfindungsgemäßen Verfahrens (a) als Ausgangsstoffe benötigten Anhydride sind durch die Formel (II) allgemein definiert. In dieser Formel (II) steht $A^2$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Anhydride der Formel (II) sind allgemein bekannte Verbindungen der organischen Chemie oder erhältlich in Analogie zu allgemein bekannten Verfahren (vgl. z.B. Gazz. chim. ital. 57, 300-311 [1927]).

Die zur Durchführung des erfindungsgemäßen Verfahrens (a) weiterhin als Ausgangsstoffe benötigten Amine sind durch die Formel (III) allgemein definiert. In dieser Formel (III) stehen $R^1$ und $A^1$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind teilweise bekannt (vgl. z.B. J. org. Chem. 49, 4295-97 [1984]; DE-OSn 2 823 168, 2 848 531, 2 932 689, 3 223 505 und 3 431 219 sowie J. Chem. Soc. B, 1971, 1231-1240).

Noch nicht bekannt sind Amine der Formel (IIIa),

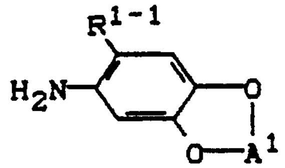 (IIIa)

in welcher
$R^{1-1}$ für Fluor steht und
$A^1$ für einen Rest der Formel

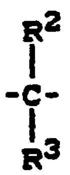 ; 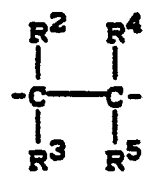 oder 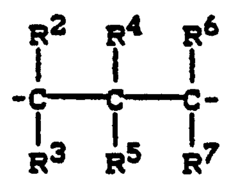 steht,

wobei
$R^2$, $R^3$, $R^4$, $R^5$, $R^6$ und $R^7$ unabhängig voneinander jeweils für Wasserstoff, Halogen, Alkyl oder Halogenalkyl stehen.

Bevorzugt sind Verbindungen der Formel (IIIa), bei welchen
$R^{1-1}$ für Fluor steht und
$A^1$ für einen Rest der Formel

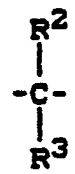 ; 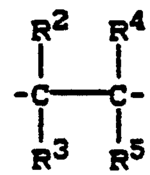 oder 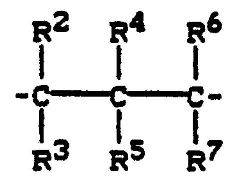 steht, wobei

$R^2$, $R^3$, $R^4$, $R^5$, $R^6$ und $R^7$ unabhängig voneinander jeweils für Wasserstoff, Fluor, Chlor, Brom oder für jeweils geradkettiges oder verzweigtes Alkyl oder Halogenalkyl mit jeweils 1 bis 3 Kohlenstoffatomen und

im Fall des Halogenalkyl mit 1 bis 7 gleichen oder verschiedenen Halogenatomen - insbesondere Fluor, Chlor oder Brom - stehen.

In der Formel (IIIa) steht $A^1$ besonders bevorzugt bzw. ganz besonders bevorzugt für diejenigen Reste, die bereits bei der Beschreibung der erfindungsgemäßen Stoffe der Formel (I) als besonders bevorzugt bzw. ganz besonders bevorzugt für diesen Substituenten genannt wurden.

Man erhält sie in Analogie zu bekannten Verfahren, beispielsweise, wenn man Nitroverbindungen der Formel (Va),

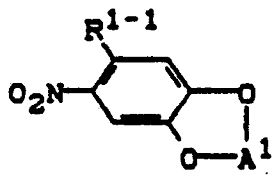

(Va)

in welcher
$R^{1-1}$ und $A^1$ die oben angegebene Bedeutung haben,
mit üblichen Reduktionsmitteln wie beispielsweise molekularem Wasserstoff in Gegenwart eines Katalysators wie beispielsweise Raney-Nickel gegebenenfalls in Gegenwart eines Verdünnungsmittels wie beispielsweise Tetrahydrofuran bei Temperaturen zwischen 0 °C und 80 °C und einem Druck zwischen 20 und 60 bar in allgemein üblicher Art und Weise reduziert.

Die Nitroverbindungen der Formel (Va) sind teilweise bekannt (vgl. z.B. Jpn. Kokai Tokkyo Koho JP 60/48 983).

Noch nicht bekannt sind Nitroverbindungen der Formel (Vb),

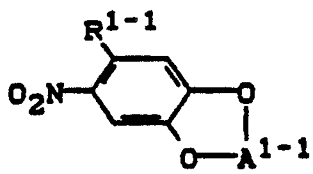

(Vb)

bei welchen
$R^{1-1}$ für Fluor steht und
$A^{1-1}$ für einen Rest der Formel

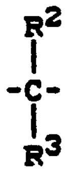 , 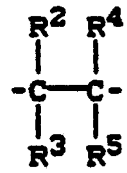 oder 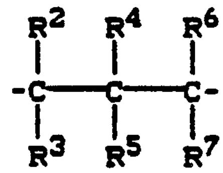 steht, wo

$R^2$, $R^3$, $R^4$, $R^5$, $R^6$ und $R^7$ unabhängig voneinander jeweils für Wasserstoff, Halogen, Alkyl oder Halogenalkyl stehen, und wobei $A^{1-1}$ jedoch nicht für den Ethandiyl-Rest steht.

Bevorzugt sind Verbindungen der Formel (Vb), bei welchen
$R^{1-1}$ für Fluor steht und
$A^{1-1}$ für einen Rest der Formel

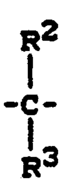 ; 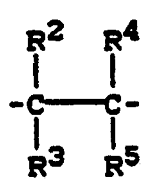 oder 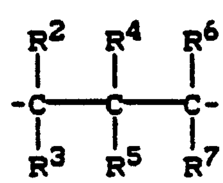 steht,

wobei
$R^2$, $R^3$, $R^4$, $R^5$, $R^6$ und $R^7$ unabhängig voneinander jeweils für Wasserstoff, Fluor, Chlor, Brom oder für

jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen - insbesondere Fluor, Chlor oder Brom - stehen und wobei

$A^{1-1}$ jedoch nicht für den Ethandiyl-Rest steht.

In der Formel (Vb) steht $A^{1-1}$ besonders bevorzugt bzw. ganz besonders bevorzugt für diejenigen Reste, die bereits im Zusammenhang mit der Beschreibung der erfindungsgemäßen Stoffe der Formel (I) als besonders bevorzugt bzw. ganz besonders bevorzugt für diesen Substituenten genannt wurden, mit Ausnahme des 1,2-Ethandiyl-restes.

Man erhält sie in Analogie zu bekannten Verfahren, beispielsweise, wenn man Halogenaromaten der Formel (VIb),

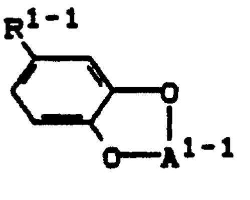

(VIb)

in welcher

$R^{1-1}$ und $A^{1-1}$ die oben angegebene Bedeutung haben,

mit einem üblichen Nitrierungsmittel wie beispielsweise Salpetersäure gegebenenfalls in Gegenwart eines Katalysators wie beispielsweise Schwefelsäure bei Temperaturen zwischen - 20 °C und + 30 °C nitriert.

Die Halogenaromaten der Formel (VIb) sind teilweise bekannt (vgl. z.B. Khim. Geterotsikl. Soedin. 1978, 1465-1471 bzw. CA 90: 121 506 m; Doklady Akad. Nauk. S.S.S.R. 135, 377-380 [1960] bzw. CA 55: 24 238e) oder können in Analogie zu bekannten Verfahren erhalten werden (vgl. z.B. DE-OS 3 409 438; DE-OS 2 819 788; DE-OS 2 318 273 oder Tetrahedron Letters 1978, 1059-1062; sowie die Herstellungsbeispiele).

Die zur Durchführung der erfindungsgemäßen Verfahren (b) und (c) als Ausgangsstoffe benötigten N-Arylstickstoffheterocyclen sind durch die Formel (Ia) allgemein definiert. In dieser Formel (Ia) stehen $R^1$ und $A^1$ vorzugsweise für diejenigen Reste, die bereits im Zusammenhang mit der Beschreibung der erfindungsgemäßen Stoffe der Formel (I) als bevorzugt für diese Substituenten genannt wurden.

$Het^1$ steht vorzugsweise für einen Rest

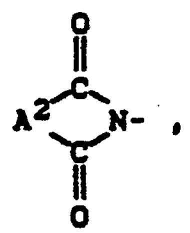

,

wobei $A^2$ vorzugsweise für diejenigen Reste steht, die bereits im Zusammenhang mit der Beschreibung der erfindungsgemäßen Stoffe der Formel (I) als bevorzugt für diesen Substituenten genannt wurden.

Die N-Arylstickstoffheterocyclen sind erfindungsgemäße Verbindungen und erhältlich mit Hilfe des erfindungsgemäßen Verfahrens (a).

Das zur Durchführung des erfindungsgemäßen Verfahrens (c) weiterhin als Ausgangsstoff benötigte "Lawesson Reagenz" ist durch die Formel (IV) definiert.

Das "Lawesson Reagenz" der Formel (IV) ist bekannt (vgl. z.B. DE-OS 2 606 083; Bull. Soc. Chim. Belg. 87, 223-228 [1978]).

Die zur Durchführung des erfindungsgemäßen Verfahrens (d) als Ausgangsstoffe benötigten N-Aryl-Stickstoffheterocyclen sind durch die Formel (Ib) allgemein definiert. In dieser Formel (Ib) stehen $R^1$ und $A^1$ vorzugsweise für diejenigen Reste, die bereits im Zusammenhang mit der Beschreibung der erfindungsgemäßen Stoffe der Formel (I) als bevorzugt für diese Substituenten genannt wurden.

$Het^2$ steht vorzugsweise für einen Rest

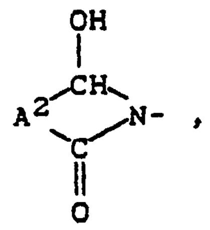

wobei $A^2$ vorzugsweise für diejenigen Reste steht, die bereits im Zusammenhang mit der Beschreibung der erfindungsgemäßen Stoffe der Formel (I) als bevorzugt für diesen Substituenten genannt wurden.

Die N-Aryl-Stickstoffheterocyclen der Formel (Ib) sind erfindungsgemäße Verbindungen und erhältlich mit Hilfe des erfindungsgemäßen Verfahrens (b).

Die zur Durchführung des erfindungsgemäßen Verfahrens (e) als Ausgangsstoffe benötigen N-Aryl-Stickstoffheterocyclen sind durch die Formel (Id) allgemein definiert. In dieser Formel (Id) stehen $R^1$ und $A^1$ vorzugsweise für diejenigen Reste, die bereits im Zusammenhang mit der Beschreibung der erfindungsgemäßen Stoffe der Formel (I) als bevorzugt für diese Substituenten genannt wurden.

$Het^4$ steht vorzugsweise für einen Rest

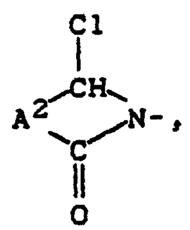

wobei $A^2$ vorzugsweise für diejenigen Reste steht, die bereits im Zusammenhang mit der Beschreibung der erfindungsgemäßen Stoffe der Formel (I) als bevorzugt für diesen Substituenten genannt wurden.

Die N-Aryl-Stickstoffheterocyclen der Formel (Id) sind erfindungsgemäße Verbindungen und erhältlich mit Hilfe des erfindungsgemäßen Verfahrens (d).

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Carbonsäuren, wie Essigsäure oder Propionsäure.

Das erfindungsgemäße Verfahren (a) gegebenenfalls in Gegenwart eines geeigneten Reaktionshilfsmittels durchgeführt werden. Vorzugsweise verwendet man anorganische oder organische Säuren wie beispielsweise Essigsäure oder p-Toluolsulfonsäure, Anhydride wie beispielsweise Acetanhydrid oder Säurechloride wie Acetylchlorid als Reaktionshilfsmittel. Es ist auch möglich andere übliche wasserabspaltende Mittel wie beispielsweise N,N′-Dicyclohexylcarbodiimid oder übliche Acylierungskatalysatoren, wie beispielsweise 4-(N,N-Dimethylamino)-pyridin als Reaktionshilfsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 180 °C, vorzugsweise bei Temperaturen zwischen 50 °C und 150 °C.

Zur Durchführung des erfindungsgemäßen Verfahrens (a) setzt man pro Mol an Anhydrid der Formel (II) im allgemeinen 1.0 bis 1.5 Mol, vorzugsweise 1.0 bis 1.2 Mol an Amin der Formel (III) und gegebenenfalls 0.01 bis 1.2 Mol, vorzugsweise 0.1 bis 1.0 Mol an Reaktionshilfsmittel ein. Die Reaktionsdurchführung, Aufarbeitung und Isolierung der Reaktionsprodukte erfolgt nach allgemein üblichen Methoden (vgl. auch die Herstellungsbeispiele).

Als Reduktionsmittel zur Durchführung des erfindungsgemäßen Verfahrens (b) kommen alle üblichen für derartige Reduktionsreaktionen verwendbaren Reduktionsmittel infrage. Vorzugsweise verwendet man komplexe Hydride, wie beispielsweise Natriumborhydrid, Natriumcyanoborhydrid, Lithiumborhydrid oder Lithiumaluminiumhydrid.

Als Verdünnungsmittel zur Durchführung des erfindungsgemäßen Verfahrens (b) kommen in Abhängigkeit vom verwendeten Reduktionsmittel alle üblichen organischen oder anorganischen Lösungsmittel infrage. Vorzugsweise verwendet man Ether, wie Diethylether, Dioxan oder Tetrahydrofuran oder Alkohole, wie Methanol, Ethanol oder Propanol.

Die Reaktionstemperaturen können bei der Durchführung des erfindungsgemäßen Verfahrens (b) in Abhängigkeit vom verwendeten Reduktionsmittel in einem größeren Bereich variiert werden. Im allgemeinen arbeitet man bei Temperaturen zwischen - 20 °C und + 150 °C, vorzugsweise bei Temperaturen zwischen 0 °C und 120 °C.

Zur Durchführung des erfindungsgemäßen Verfahrens (b) setzt man pro Mol an N-Aryl-Stickstoffheterocyclus der Formel (Ia) im allgemeinen 0.1 bis 2.0 Mol, vorzugsweise 0.25 bis 1.5 Mol an Reduktionsmittel ein.

Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether.

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 180 °C, vorzugsweise bei Temperaturen zwischen 20 °C und 150 °C.

Zur Durchführung des erfindungsgemäßen Verfahrens (c) setzt man pro Mol an N-Aryl-Stickstoffheterocyclus der Formel (Ia) im allgemeinen 0.2 bis 2.0 Mol, vorzugsweise 0.5 bis 1.5 Mol an "Lawesson Reagenz" der Formel (IV) ein.

Die Reaktionsdurchführung, Aufarbeitung und Isolierung der Reaktionsprodukte erfolgt in Analogie zu bekannten Verfahren (vgl. z.B. Bull. Soc. Chim. Belg. 87, 223-228 [1978] sowie die Herstellungsbeispiele).

In der Regel erhält man bei der Durchführung des erfindungsgemäßen Verfahrens (c) als Endprodukt ein Gemisch aus Verbindungen der Formel (Ic1),

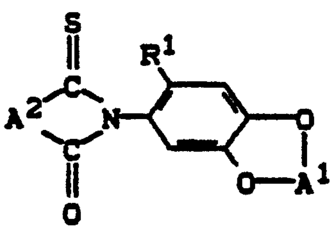

(Ic1)

und der Formel (Ic2),

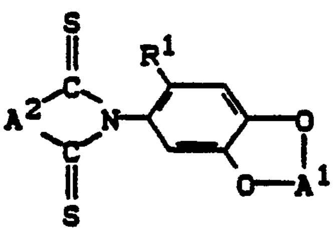

(Ic2)

wobei

$A^1$, $A^2$ und $R^1$ in beiden Formeln die oben angegebenen Bedeutungen haben.

Diese Gemische lassen sich mit üblichen Trennmethoden (Chromatographie, Kristallisation) auftrennen.

Als Verdünnungsmittel zur Durchführung des erfindungsgemäßen Verfahrens (d) kommen inerte organische Lösungs mittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Nitrile wie Acetonitril oder

Propionitril.

Das erfindungsgemäße Verfahren (d) kann gegebenenfalls in Gegenwart eines geeigneten Reaktionshilfsmittels durchgeführt werden. Als solche kommen insbesondere organische Amine oder Amide infrage.Vorzugsweise verwendet man Pyridin, Dimethylanilin oder Dimethylformamid.

Die Reaktionstemperaturen können bei der Durchführung des erfindungsgemäßen Verfahrens (d) in einem größeren Bereich variiert werden. Im allgemeinen arbeitet man bei Temperaturen zwischen 0 °C und 120 °C, vorzugsweise bei Temperaturen zwischen 20 °C und 80 °C.

Zur Durchführung des erfindungsgemäßen Verfahrens (d) setzt man pro Mol an N-Aryl-Stickstoffheterocyclus der Formel (Ib) im allgemeinen 1.0 bis 5.0 Mol, vorzugsweise 1.0 bis 2.0 Mol an Thionylchlorid und gegebenenfalls 0.1 bis 2.0 Mol, vorzugsweise 0.5 bis 1.5 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Als Katalysatoren zur Durchführung des erfindungsgemäßen Verfahrens (e) kommen alle üblichen Hydrierkatalysatoren infrage. Vorzugsweise verwendet man Edelmetallkatalysatoren, wie beispielsweise Platin, Platinoxid, Palladium oder Ruthenium gegebenenfalls auf einem geeignetem Träger wie beispielsweise Kohlenstoff oder Siliciumdioxid.

Als Verdünnungsmittel zur Durchführung des erfindungsgemäßen Verfahrens (e) kommen inerte organische Lösungsmittel infrage. Hierzu gehören insbesondere aliphatische oder alicyclische gegebenenfalls halogenierte Kohlenwasserstoffe, wie beispielsweise Benzin, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Alkohole wie Methanol, Ethanol oder Propanol.

Das erfindungsgemäße Verfahren (e) kann gegebenenfalls in Gegenwart eines geeigneten Säurebindemittels durchgeführt werden. Vorzugsweise verwendet man Alkalimetallcarbonate wie Natriumcarbonat oder Kaliumcarbonat oder organische Basen wie Pyridin oder Lutidin.

Die Reaktionstemperaturen können bei der Durchführung des erfindungsgemäßen Verfahrens (e) in einem größeren Bereich variiert werden. Im allgemeinen arbeitet man bei Temperaturen zwischen - 20 °C und 100 °C, vorzugsweise bei Temperaturen zwischen 0 °C und 50 °C.

Das erfindungsgemäße Verfahren kann bei Normaldruck oder unter erhöhtem Druck durchgeführt werden. Vorzugsweise arbeitet man unter Normaldruck.

Zur Durchführung des erfindungsgemäßen Verfahrens (e) setzt man pro Mol an N-Aryl-Stickstoffheterocyclus der Formel (Id) im allgemeinen 1.0 bis 3.0 Mol, vorzugsweise 1.0 bis 1.5 Mol an Wasserstoff und gegebenenfalls 1.0 bis 3.0 Mol, vorzugsweise 1.0 bis 1.5 Mol an Säurebindemittel ein.

Die Reaktionsdurchführung, Aufarbeitung und Isolierung der Reaktionsprodukte erfolgt nach allgemein üblichen Methoden (vgl. auch die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo ie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-. Bananen-, Kaffee-, Tee-, Gummi- Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung von dikotylen Unkräutern insbesondere in monokotylen Kulturen, wie beispielsweise Gerste, Weizen oder Mais einsetzen.

Darüberhinaus greifen die erfindungsgemäßen Wirkstoffe in den Metabolismus der Pflanzen eine und können deshalb als Wachstumsregulatoren eingesetzt werden.

Für die Wirkungsweise von Pflanzenwachstumsregulatoren gilt nach der bisherigen Erfahrung, daß eine Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also EMulgiermitteln und/oder Dispergiermitteln und/oder schaumerzeugenden Mitteln.

Im Falle der Benutzung von Wasser als Streckmittel können z.B. auch organische Lösungsmitel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenswasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage:

z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat; sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalooyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N′-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekäm pfung in Sojabohnen, infrage. Auch Mischungen mit

2,4-Dichlorphenoxyessigsäure;
2,4-Dichlorphenoxypropionsäure;
4-(2,4-Dichlorphenoxy)-buttersäure;
(2-Methyl-4-chlorphenoxy)-essigsäure;
4-(4-Chlor-2-methyl)-phenoxybuttersäure;
3,5,6-Trichlor-2-pyridyloxyessigsäure;
2-[4-(2,4-Dichlorphenoxy)-phenoxy]-propionsäure, deren Methyl- oder deren Ethylester;
2-{4-[(6-Chlor-2-benzoxazolyl)-oxy]-phenoxy}-propansäure, deren Methyl- oder deren Ethylester;
2-[4-(3,5-Dichlorpyrid-2-yloxy)-phenoxy]-propionsäure-(trimethylsilylmethylester);
3,6-Dichlor-2-pyridincarbonsäure;
N-(1-Ethylpropyl)-3,4-dimethyl-2,6-dinitroanilin;
Methyl-5-(2,4-dichlorphenoxy)-2-nitrobenzoat;
N,N-Dimethyl-N′-(3-chlor-4-methylphenyl)-harnstoff;
N,N-Dimethyl-N′-(4-isopropylphenyl)-harnstoff;
Methyl-6,6-dimethyl-2,4-dioxo-3-[1-(2-propenyloxyamino)butyliden]-cyclohexancarbonsäure;
Methyl-2-[4,5-dihydro-4-methyl-4-(1-methylethyl)-5-oxo-1H-imidazol-2-yl]-4(5)-methylbenzoat;
3,5-Dibrom-4-hydroxy-benzonitril;
3,5-Diiod-4-hydroxybenzonitril;
N-Methyl-2-(1,3-benzthiazol-2-yloxy)-acetanilid;
2-[[[[(4,6-Dimethoxypyrimidin-2-yl)-amino]-carbonyl]-amino]-sulfonyl]-methyl]-benzoesäuremethylester;
2-Chlor-N-{[(4-methoxy-6-methyl-1,3,5-triazin-2-yl)-amino]-carbonyl}-benzolsulfonamid;
2-{[[((4-Methoxy-6-methyl-1,3,5-triazin-2-yl)-amino)carbonyl]-amino]-sulfonyl}-benzoesäure oder deren Methylester;
3-[[[[(4-Methoxy-6-methyl-1,3,5-triazin-2-yl)-amino]carbonyl]-amino]-sulfonyl]-thiophen-2-carbonsäuremethylester;
N,N-Diisoproyl-S-(2,3,3-trichlorallyl)-thiolcarbamat;
4-Ethylamino-2-t-butylamino-6-methylthio-s-triazin und 3-Isopropyl-2,1,3-benzothiadiazin-4-on-2,2-dioxid sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoff können sowohl vor als auch nach dem Auflaufen der Pflanzen appliziert werden.

Sie können auch vor der Saat in den Boden eingearbeitet werden.

Die angewandte Wirkstoffmenge kann bei der Anwendung als Herbizide in einem größeren Bereich

schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die erfindungsgemäßen Wirkstoffe können bei der Anwendung als Wachstumregulatoren in den Formulierungen ebenfalls in Mischung mit anderen bekannten Wirkstoffen vorliegen, wie Fungizide, Insektizide, Akarizide und Herbizide sowie in Mischungen mit Düngemitteln und anderen Wachstumsregulatoren.

Die Wirkstoffe können dabei als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können bei der Anwendung als Wachstumsregulatoren ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß Anwendung der Wachstumsregulatoren in einem bevorzugten Zeitraum vorgenommen wird, dessen genaue Abgrenzung sich nach den klimatischen und vegetativen Gegebenheiten richtet.

## Herstellungsbeispiele

### Beispiel 1

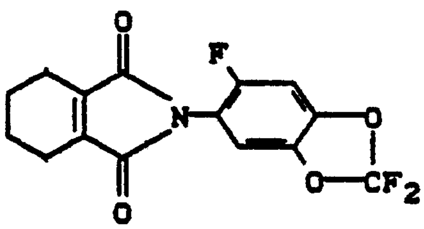

**(Verfahren a)**

4 g (0,026 Mol) 3,4,5,6-Tetrahydrophthalsäureanhydrid und 5 g (0,025 Mol) 2-Fluor-4,5-dioxydifluormethylenanilin in 50 ml Eisessig werden 5 Stunden bei 80 °C gerührt. Die erkaltete Reaktionsmischung wird in 250 ml Wasser gegeben, der so entstandene Niederschlag abgesaugt, gewaschen und aus Ethanol umkristallisiert.

Man erhält 6,3 g (74 % der Theorie) an N-(2-Fluor-4,5-dioxydifluormethylen-phenyl)-3,4,5,6-tetrahydrophthalimid vom Schmelzpunkt 90 °C - 92 °C.

Beispiel 2

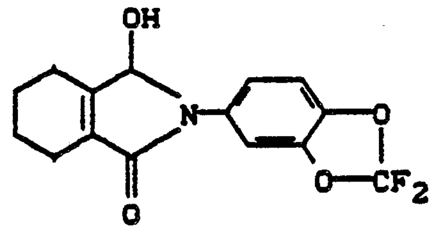

(Verfahren b)

4 g (0,013 Mol) N-(3,4-Dioxydifluormethylenphenyl)-3,4,5,6-tetrahydrophthalimid in 100 ml Methanol werden mit 0,75 g (0,02 Mol) Natriumborhydrid versetzt, 2 Stunden bei Raumtemperatur gerührt, in 200 ml Eiswasser gegeben, der so entstandene Niederschlag abgesaugt, gewaschen und getrocknet.

Man erhält 3,7 g (92 % der Theorie) an N-(3,4-Dioxydifluormethylenphenyl)-3-hydroxy-2,3,4,5,6,7-hexahydroisoindol-1-on vom Schmelzpunkt 190 °C - 192 °C.

Beispiel 3

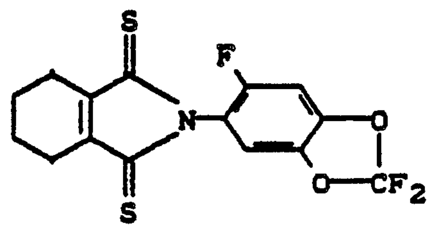

(Verfahren c)

2,28 g (0,007 Mol) N-(2-Fluor-4,5-dioxydifluormethylenphenyl)-3,4,5,6-tetrahydrophthalimid und 2,96 (0,0074 Mol) 2,4-Bis(4-methoxyphenyl)-1,3-dithia-2,4-diphosphetan-2,4-disulfid werden in 10 ml absolutem Toluol 1 Stunde unter Rückfluß erhitzt, auf Raumtemperatur abgekühlt, mit Wasser gewaschen, über Magnesiumsulfat getrocknet und im Vakuum eingeengt. Der ölige Rückstand wird durch Chromatographie an Kieselgel (Laufmittel : Cyclohexan/Essigester 6 : 1) gereinigt und aus Ether/n-Hexan kristallisiert.

Man erhält 0,68 g (27,2 % der Theorie) an N-(2-Fluor-4,5-dioxydifluormethylen-phenyl)-3,4,5,6-dithiotetrahydrophthalimid vom Schmelzpunkt 107 °C.

Beispiel 4

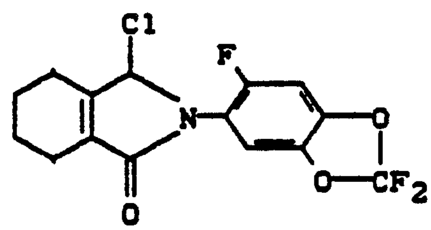

(Verfahren d)

6 g (0,018 Mol) N-(4,5-Dioxydifluormethylen-2-fluorphenyl)-3-hydroxy-2,3,4,5,6,7-hexahydroisoindol-1-on in 30 ml Chloroform werden mit 3,3 g (0,028 Mol) Thionylchlorid versetzt und langsam auf 50 °C erwärmt.

Nach beendeter Gasentwicklung rührt man eine weitere Stunde bei 50 °C und engt dann im Vakuum ein.

Man erhält 6,2 g (100 % der Theorie) an N-(4,5-Dioxydifluormethylen-2-fluor-phenyl)-3-chlor-2,3,4,5,6,7-hexahydroisoindol-1-on als Öl.

$^1$H-NMR ($CDCl_3$) : $\delta$ =
1,70 (m, 4H); 2,30 (m, 3H);
2,59 (m, 1H); 6,20 (s, 1H);
6,98 (d, 1H); 7,21 (d, 1H) ppm.

Beispiel 5

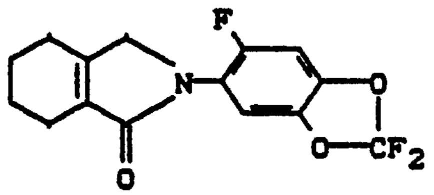

(Verfahren e)

Zu 6 g (0,017 Mol) N-(2-Fluor-4,5-dioxydifluormethylenphenyl)-3-chlor-2,3,4,5,6,7-hexahydroisoindol-1-on in 50 ml trockenem Tetrahydrofuran gibt man nacheinander 2,0 g (0,018 Mol) Lutidin und 3 g Palladium auf Aktivkohle (5 %ig) und hydriert die Mischung anschließend mit Wasserstoff unter Normaldruck. Nach beendeter Wasserstoffaufnahme wird der Katalysator abfiltriert, das Filtrat im Vakuum eingeengt und über eine kurze Kieselgelsäule filtriert. Danach wird das Lösungsmittel im Vakuum entfernt, der Rückstand durch Verrühren mit n-Hexan zur Kristallisation gebracht, abgesaugt und getrocknet.

Man erhält 2,8 g (52 % der Theorie) an N-(2-Fluor-4,5-dioxydifluormethylen-phenyl)-2,3,4,5,6,7-hexahydroisoindol-1-on vom Schmelzpunkt 105 °C -106 °C.

In entsprechender Weise und gemäß den allgemeinen Angaben zur Herstellung erhält man die folgenden N-ArylStickstoffheterocyclen der allgemeinen Formel (I):

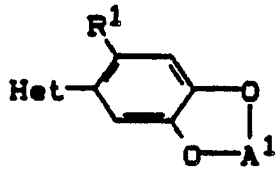

(I)

## Tabelle 1

| Bsp. Nr. | Het | $R^1$ | $A^1$ | physikalische Eigenschaften |
|---|---|---|---|---|
| 6 | 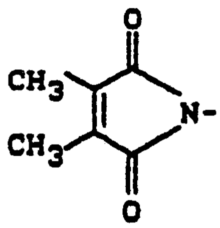 | F | $-CF_2-$ | Fp 118 °C |
| 7 | 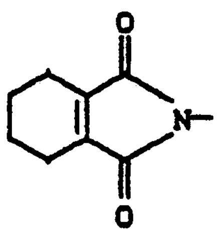 | F | $-CF_2-CHF-$ | Fp 120 °C |
| 8 | 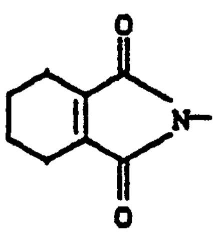 | F | $-CHF-CF_2-$ | Fp 145 °C |

Tabelle 1 (Fortsetzung)

| Bsp. Nr. | Het | $R^1$ | $A^1$ | physikalische Eigenschaften |
|---|---|---|---|---|
| 9 | 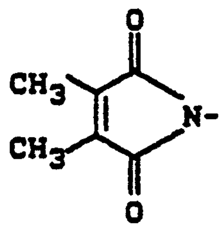 | F | $-CHF-CF_2-$ | Fp 88 °C |
| 10 | 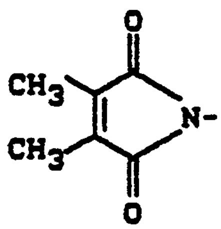 | F | $-CF_2-CHF-$ | Fp 103 °C |
| 11 | 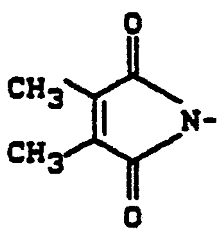 | Cl | $-CF_2-$ | Fp 109 °C |
| 12 | 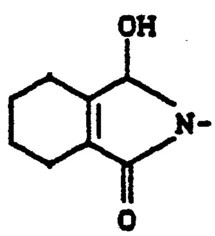 | F | $-CF_2-$ | Fp 161-162°C |
| 13 | 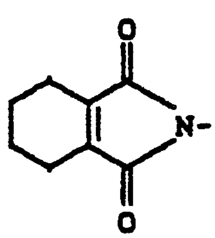 | F | $-CF_2-CF_2-$ | Fp 124 °C |

<u>T a b e l l e   1</u> (Fortsetzung)

| Bsp. Nr. | Het | $R^1$ | $A^1$ | physikalische Eigenschaften |
|---|---|---|---|---|
| 14 | 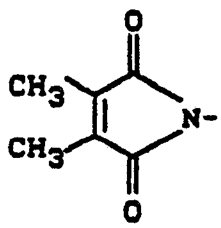 | F | $-CF_2-CF_2-$ | Fp 93 °C |
| 15 | 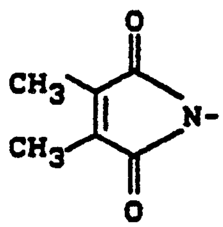 | H | 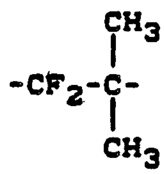 | Fp 107 °C |
| 16 | 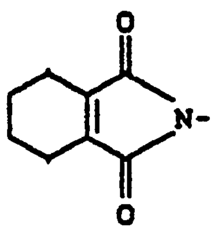 | F | $-CH_2-CH_2-$ | Fp 110 °C |
| 17 | 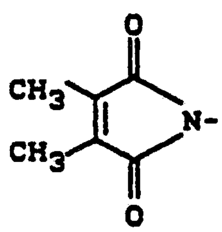 | F | $-CH_2-CH_2$ | Fp 146 °C |
| 18 | 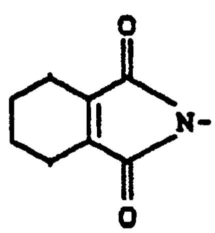 | H | $-CF_2-$ | Fp 163 °C |

**Tabelle 1** (Fortsetzung)

| Bsp. Nr. | Het | $R^1$ | $A^1$ | physikalische Eigenschaften |
|---|---|---|---|---|
| 19 | 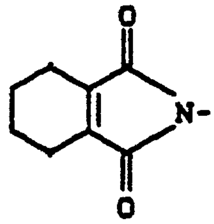 | H | $-CF_2-CF_2-$ | Fp 138,5 °C |
| 20 | 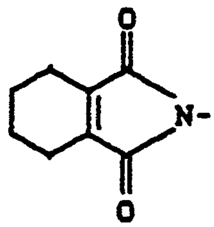 | Cl | $-CHF-CF_2-$ | Fp 130 °C |
| 21 | 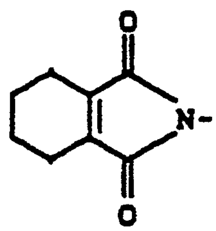 | H | $-CH_2-CH_2-$ | Fp 168 °C |
| 22 | 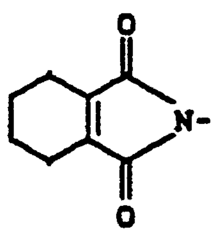 | H | $-CH_2-$ | Fp 143 °C |
| 23 | 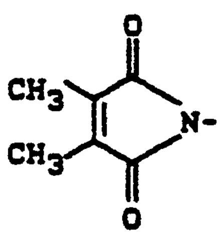 | H | $-CH_2-CH_2-$ | Fp 164 °C |

**Tabelle 1** (Fortsetzung)

| Bsp. Nr. | Het | $R^1$ | $A^1$ | physikalische Eigenschaften |
|---|---|---|---|---|
| 24 | 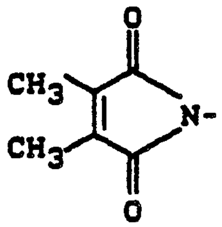 | H | $-CH_2-$ | Fp 150 °C |
| 25 | 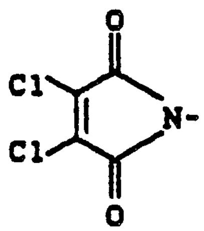 | H | $-CH_2-CH_2-$ | Fp 172 °C |
| 26 | 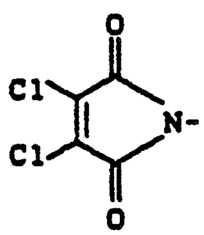 | H | $-CH_2-$ | Fp 180-183° C |
| 27 | 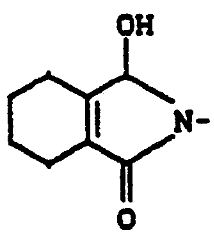 | H | $-CH_2-CH_2-$ | Fp 195 °C |
| 28 | 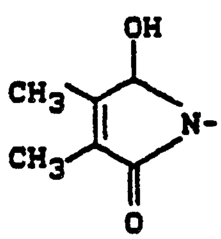 | H | $-CH_2-CH_2-$ | Fp 162 °C |

**Tabelle 1** (Fortsetzung)

| Bsp. Nr. | Het | $R^1$ | $A^1$ | physikalische Eigenschaften |
|---|---|---|---|---|
| 29 | 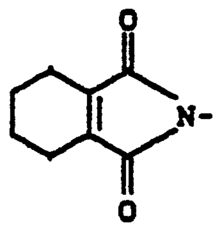 | Cl | $-CF_2-$ | Fp 87 °C |
| 30 | 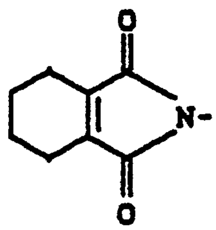 | H | $-CF_2-C(CH_3)_2-$ | Fp 120 °C |
| 31 | 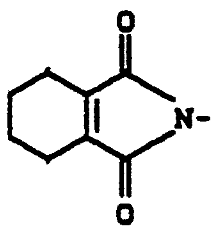 | H | $-CH_2-CF_2-$ | Fp 107 °C |

Herstellung der Ausgangsverbindungen

Beispiel III-1

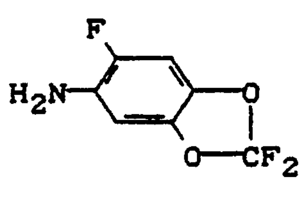

230 g (1,04 Mol) 5-Nitro-2,2,6-trifluor-1,3-benzodioxol und 20 g Raney-Nickel in 800 ml Tetrahydrofuran werden bei 20 °C bis 45 °C und 30 bis 50 bar Wasserstoffdruck bis zum Ende der Wasserstoffaufnahme hydriert. Zur Aufarbeitung wird der Katalysator abfiltiert und das Filtrat destilliert.

Man erhält 179 g (90 % der Theorie) an 5-Amino-2,2,6-trifluor-1,3-benzodioxol vom Siedepunkt 86 °C - 87 °C bei 16 mbar und vom Brechungsindex $n_D^{20}$ 1,4812.

In entsprechender Weise und gemäß den allgemeinen Angaben zur Herstellung erhält man die folgenden Amine der allgemeinen Formel (III):

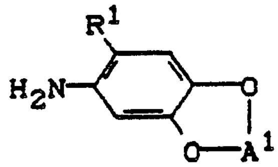

(III)

**Tabelle 2**

| Bsp. Nr. | $R^1$ | $A^1$ | Brechungsindex/ Siedepunkt |
|---|---|---|---|
| III-2 | F | $-CF_2-CF_2-$ | $n_D^{20}$ 1,4550 |
| III-3 | F | $-CFCl-CF_2-$ | $n_D^{20}$ 1,4872 |
| III-4 | F | 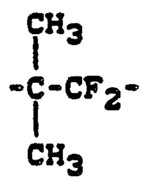 | $n_D^{20}$ 1,4970 |
| III-5 | F | $-CHF-CF_2-$ | $n_D^{20}$ 1,4852 |
| III-6 | F | $-CH_2-CH_2-$ | Kp 148-150°C/ 16 mbar |
| III-7 | Cl | $-CF_2-CF_2-$ | $n_D^{20}$ 1,4862 |
| III-8 | Cl | $-CFCl-CF_2-$ | $n_D^{20}$ 1.5170 |
| III-9 | Cl | $-CHF-CF_2-$ | $n_D^{20}$ 1,5170 |

Beispiel V-1

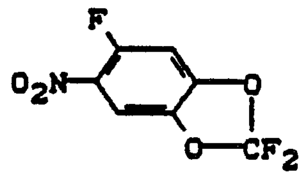

Zu einer Mischung aus 202 ml 67prozentiger Salpetersäure und 242 ml konzentrierter Schwefelsäure tropft man bei 10 °C unter Rühren 202 g (1,15 Mol) 2,2,5-Trifluor-1,3-benzodioxol (vgl. Doklady Akad. Nauk. S.S.S.R. 135, 377-380 [1960] bzw. C.A.: 55, 24 238e), rührt nach beendeter Zugabe eine Stunde bei 10 °C und eine weitere Stunde bei 20 °C, gießt die Mischung auf 600 g Eis, trennt die organische Phase ab und destilliert.

Man erhält 232 g (91,3 % der Theorie) an 5-Nitro-2,2,6-trifluor-1,3-benzodioxol vom Siedepunkt 113 °C - 114 °C bei 20 mbar und vom Brechungsindex $n_D^{20}$ 1,4968.

In entsprechender Weise und gemäß den allgemeinen Angaben zur Herstellung erhält man die folgenden Nitroverbindungen der allgemeinen Formel (V):

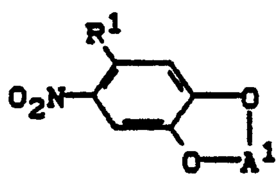

(V)

**Tabelle 3**

| Bsp. Nr. | $R^1$ | $A^1$ | physikalische Eigenschaften |
|---|---|---|---|
| V-2 | F | $-CF_2-CF_2-$ | Kp 96-98° C/ 12 mbar |
| V-3 | F | $-CHF-CF_2-$ | Kp 122-123° C/ 12 mbar |
| V-4 | F | $-CFCl-CF_2-$ | Kp 130° C/ 12 mbar |
| V-5 | F | 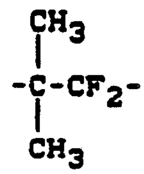 | Kp 134-138° C/ 10 mbar |
| V-6 | F | $-CH_2-CH_2-$ | Fp 134-138° C |
| V-7 | Cl | $-CF_2-CF_2-$ | Kp 106-110° C/ 10 mbar |
| V-8 | Cl | $-CFCl-CF_2-$ | Kp 100-103° C/ 12 mbar |
| V-9 | Cl | $-CHF-CF_2-$ | Kp 95-97° C/ 0,2 mbar |

Beispiel VI-1

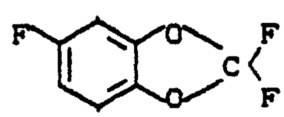

Zu 173 g (1 Mol) 5-Amino-2,2-difluor-1,3-benzodioxol (vgl. z.B. DE 2 823 168; EP 42 533) in 600 ml Wasser und 150 ml konzentrierter Salzsäure gibt man bei 0 °C 70 g (1 Mol) Natriumnitrit in 180 mol Wasser, rührt 1 Stunde bei 0 °C bis 10 °C, filtriert die Reaktionsmischung in 400 ml 30prozentige wässrige Tetrafluorborsäurelösung, saugt den ausgefallenen Niederschlag ab, trocknet bei 50 °C und 20 mbar und erwärmt das so erhältliche Diazoniumtetrafluoroborat portionsweise (in Portionen von ca. 20 bis 30 g) in einer Destillationsapparatur auf 130 °C bis 160 °C. Das Produkt destilliert dabei mit dem Gasstrom ab und

wird in einer gut gekühlten Vorlage aufgefangen. Zum Schluß steigert man die Badtemperatur auf 190 °C um die Reaktion und Destillation zu komplettieren, nimmt das überdestillierte Produkt in Dichlormethan auf, wäscht mit Wasser, trocknet über Magnesiumsulfat und destilliert ein zweites Mal über eine Kolonne.

Man erhält 89 g (50 % der Theorie) an 2,2,5-Trifluor-1,3-benzodioxol vom Siedepunkt 128 °C - 130 °C und vom Brechungsindex $n_D^{20}$ 1,4295.

In entsprechender Weise und gemäß den allgemeinen Angaben zur Herstellung erhält man die folgenden Halogenaromaten der allgemeinen Formel (VI):

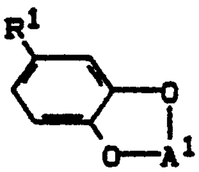

(VI)

**T a b e l l e 4**

| Bsp. Nr. | $R^1$ | $A^1$ | physikalische Eigenschaften |
|---|---|---|---|
| VI-2 | F | $-CHF-CF_2-$ | $n_D^{20}$ 1,4392 |
| VI-3 | F | $-CF_2-CF_2-$ | $n_D^{20}$ 1,4095 |
| VI-4 | F | $-CFCl-CF_2-$ | $n_D^{20}$ 1,4462 |
| VI-5 | F | $CH_3$<br>$-C-CF_2-$<br>$CH_3$ | $n_D^{20}$ 1,4572 |
| VI-6 | F | $-CH_2-CH_2-$ | $n_D^{20}$ 1,5200 |

Anwendungsbeispiele

In den folgenden Anwendungsbeispielen wurden die nachstehend aufgeführten Verbindungen als Vergleichsubstanzen eingesetzt:

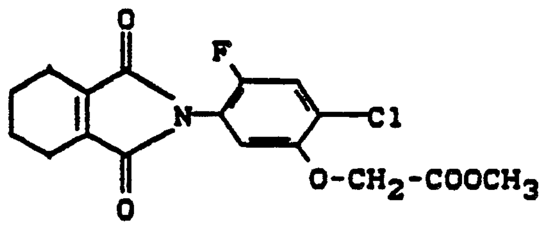

(A)

(bekannt aus EP 83 055/Verbindung Nr. 1)

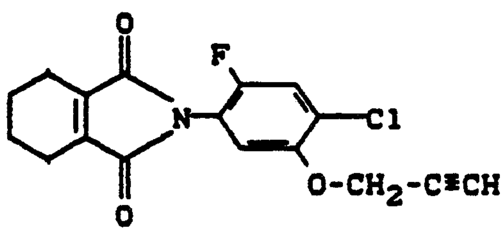

(B)

(bekannt aus EP 61 741/Verbindung Nr. 10)

Beispiel A

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Eine deutliche Überlegenheit in der herbiziden Wirksamkeit gegen Unkräuter wie beispielsweise Galinsoga, Helianthus, Polygonum oder Sinapis ebenso wie in der Nutzpflanzenselektivität gegenüber dem Stand der Technik zeigt in diesem Test z.B. die Verbindung gemäß Herstellungsbeispiel 1.

Beispiel B

Entlaubung und Austrocknung der Blätter bei Baumwolle

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

O kein Austrocknen der Blätter, kein Blattfall

\+ leichtes Austrocknen der Blätter, geringer Blattfall

\+ + starkes Austrocknen der Blätter, starker Blattfall

\+ + + sehr starkes Austrocknen der Blätter, sehr starker Blattfall

Eine deutliche Überlegenheit im Vergleich zur unbehandelten Kontrolle zeigen in diesem Test beispielsweise die Verbindungen gemäß folgender Herstellungsbeispiele: 1 und 8.

## Ansprüche

1. N-Aryl-Stickstoffheterocyclen der allgemeinen Formel (I),

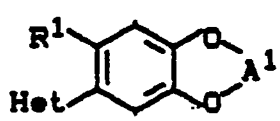

(I)

in welcher

Het für einen Heterocyclus der Formel

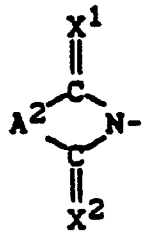

oder

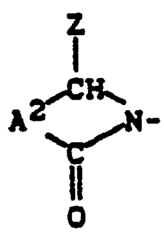

steht,

$R^1$ für Wasserstoff oder Halogen steht,

$A^1$ für einen Rest der Formel

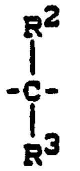 ; 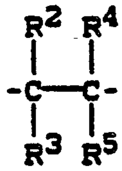 oder 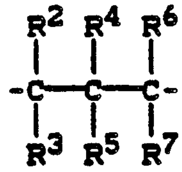 steht,

wobei
$A^2$ für einen Rest der Formel

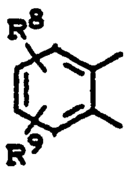 ; 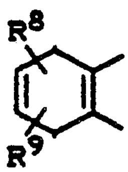 ; 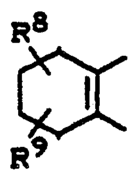 ; 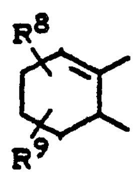 ;

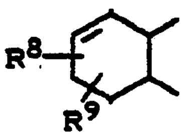 ; 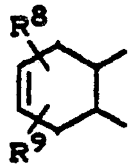 oder 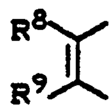 steht,

$X^1$ und $X^2$ jeweils für Sauerstoff oder Schwefel stehen,
Z für Wasserstoff, Hydroxy oder Chlor steht,
$R^2$, $R^3$, $R^4$, $R^5$, $R^6$ und $R^7$ unabhängig voneinander jeweils für Wasserstoff, Halogen, Alkyl oder Halogenalkyl stehen und
$R^8$ und $R^9$ unabhängig voneinander jeweils für Wasserstoff, Halogen, Alkyl oder Halogenalkyl stehen.

2. N-Aryl-Stickstoffheterocyclen der Formel (I) gemäß Anspruch 1,
in welcher
Het für einer Heterocyclus der Formel

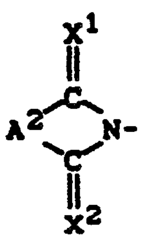 oder 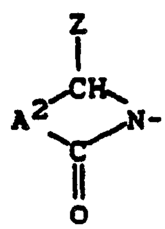 steht,

$R^1$ für Wasserstoff, Fluor, Chlor oder Brom steht und
$A^1$ für einen Rest der Formel

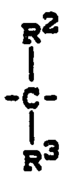 ; 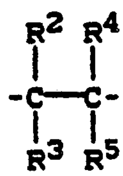 oder 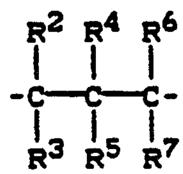 steht,

wobei
$A^2$ für einen Rest der Formel

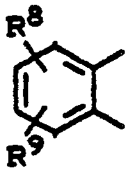 ; 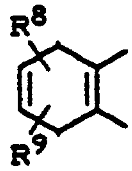 ; 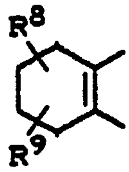 ; 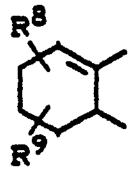 ;

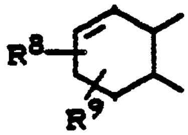 ; 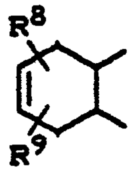 oder 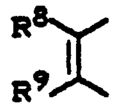 steht,

$X^1$ und $X^2$ für Sauerstoff oder Schwefel stehen,
Z für Wasserstoff, Hydroxy oder Chlor steht,
$R^2$, $R^3$, $R^4$, $R^5$, $R^6$ und $R^7$ unabhängig voneinander jeweils für Wasserstoff, Fluor, Chlor, Brom oder für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen stehen und
$R^8$ und $R^9$ unabhängig voneinander jeweils für Wasserstoff, Fluor, Chlor, Brom, oder für jeweils geradkettiges oder verzweigtes Alkyl oder Halogenalkyl mit jeweils 1 bis 3 Kohlenstoffatomen und im Fall des Halogenalkyl mit 1 bis 7 gleichen oder verschiedenen Halogenatomen stehen.

3. N-Aryl-Stickstoffheterocyclen der Formel (I) gemäß Anspruch 1,
in welcher
Het für einer Heterocyclus der Formel

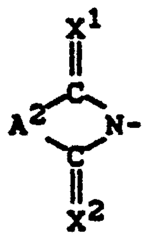 oder 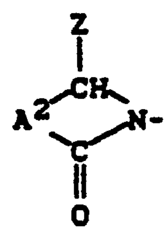 steht,

$R^1$ für Wasserstoff, Fluor oder Chlor steht und
$A^1$ für einen Rest der Formel

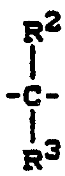 oder 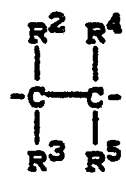 steht, wobei

$A^2$ für einen der Reste

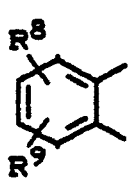 ; 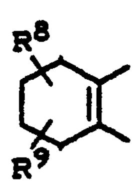 oder 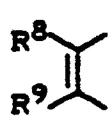 steht,

$X^1$ und $X^2$ für Sauerstoff oder Schwefel stehen,
Z für Wasserstoff, Hydroxy oder Chlor steht,
$R^2$, $R^3$, $R^4$ und $R^5$ unabhängig voneinander für Wasserstoff, Fluor, Chlor, Methyl oder Trifluormethyl stehen und

$R^8$ und $R^9$ unabhängig voneinander für Wasserstoff, Chlor, Methyl oder Trifluormethyl stehen.

4. Verfahren zur Herstellung von N-Aryl-Stickstoffheterocyclen der Formel (I),

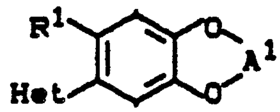 (I)

in welcher

Het für einen Heterocyclus der Formel

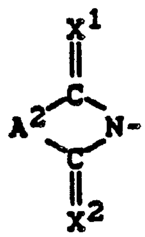 oder 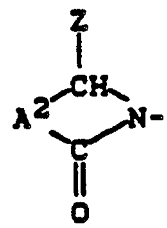 steht,

$R^1$ für Wasserstoff oder Halogen steht,

$A^1$ für einen Rest der Formel

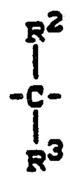 ; 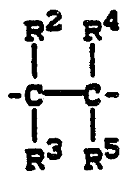 oder 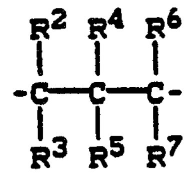 steht,

wobei

$A^2$ für einen Rest der Formel

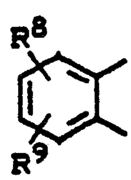 ; 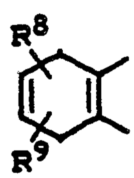 ; 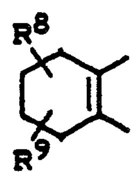 ; 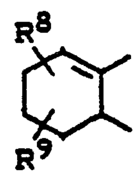 ;

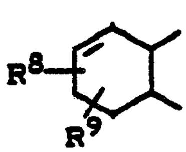 ; 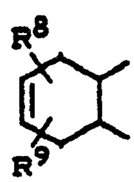 oder 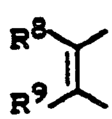 steht,

$X^1$ und $X^2$ jeweils für Sauerstoff oder Schwefel stehen,

Z für Wasserstoff, Hydroxy oder Chlor steht,

$R^2$, $R^3$, $R^4$, $R^5$, $R^6$ und $R^7$ unabhängig voneinander jeweils für Wasserstoff, Halogen, Alkyl oder Halogenalkyl stehen und

$R^8$ und $R^9$ unabhängig voneinander jeweils für Wasserstoff, Halogen, Alkyl oder Halogenalkyl stehen, dadurch gekennzeichnet, daß man

(a) N-Arylstickstoffheterocyclen der Formel (Ia),

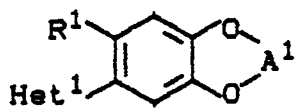

(Ia)

in welcher
Het¹ für einen Rest

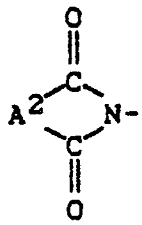

steht und
$A^1$, $A^2$ und $R^1$ die oben angegebene Bedeutung haben, erhält, wenn man Anhydride der Formel (II),

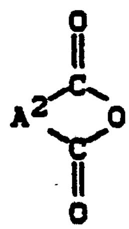

(II)

in welcher
$A^2$ die oben angegebene Bedeutung hat, mit Aminen der Formel (III),

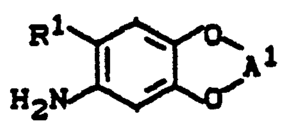

(III)

in welcher
$R^1$ und $A^1$ die oben angegebene Bedeutung haben, gegebenenfalls in Gegenwart eines Verdünnungsmittels und gegebenenfalls in Gegenwart eines Reaktionshilfsmittels umsetzt; oder daß man

(b) N-Aryl-Stickstoffheterocyclen der Formel (Ib),

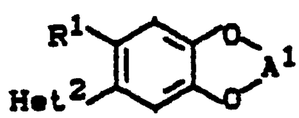

(Ib)

in welcher
$Het^2$ für einen Rest

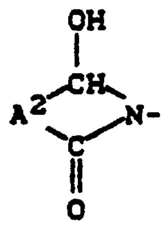

steht und
$A^1$, $A^2$ und $R^1$ die oben angegebene Bedeutung haben,
erhält, wenn man N-Aryl-Stickstoffheterocyclen der Formel (Ia),

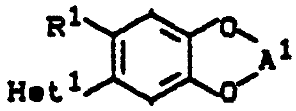

(Ia)

in welcher
$Het^1$ für eine Rest

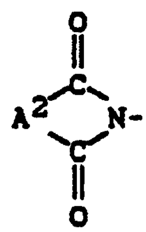

steht und
$A^1$, $A^2$ und $R^1$ die oben angegebene Bedeutung haben,
mit einem Reduktionsmittel, gegebenenfalls in Gegenwart eines Verdünnungsmittels umsetzt; oder daß man
(c) N-Aryl-Stickstoffheterocyclen der Formel (Ic),

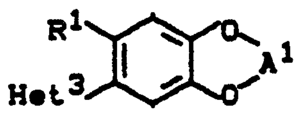

(Ic)

in welcher
$Het^3$ für einen Rest

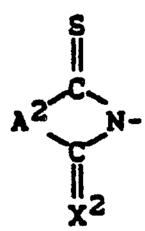

steht und
$A^1$, $A^2$, $R^1$ und $X^2$ die oben angegebene Bedeutung haben,
erhält, wenn man N-Aryl-Stickstoffheterocyclen der Formel (Ia),

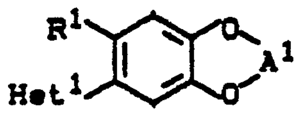

(Ia)

in welcher
Het¹ für einen Rest

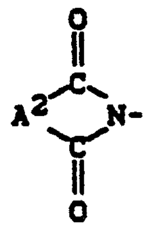

steht und
$A^1$, $A^2$ und $R^1$ die oben angegebene Bedeutung haben,
mit "Lawessons Reagenz" der Formel (IV),

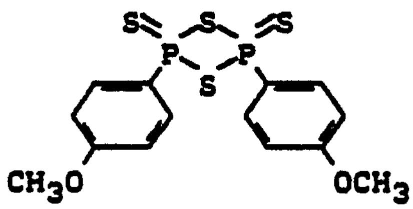

(IV)

gegebenenfalls in Gegenwart eines Verdünnungsmittels umsetzt; oder daß man
(d) N-Aryl-Stickstoffheterocyclen der Formel (Id)

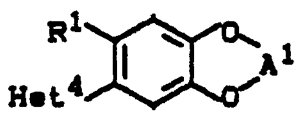

(Id)

in welcher
$Het^4$ für einen Rest

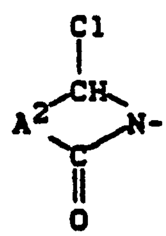

steht und
$A^1$, $A^2$ und $R^1$ die oben angegebene Bedeutung haben,
erhält, wenn man N-Aryl-Stickstoffheterocyclen der Formel (Ib),

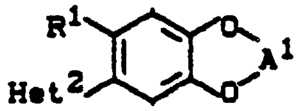

(Ib)

in welcher
Het² für einen Rest

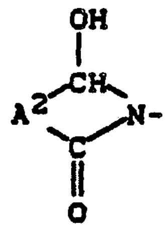

steht und
A¹, A² und R¹ die oben angegebene Bedeutung haben,
mit Thionylchlorid gegebenenfalls in Gegenwart eines Verdünnungsmittels und gegebenenfalls in Gegenwart eines Reaktionshilfsmittels umsetzt; oder daß man

(e) N-Aryl-Stickstoffheterocyclen der Formel (Ie),

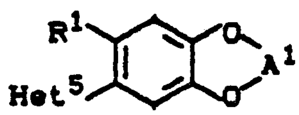

(Ie)

in welcher
Het⁵ für einen Rest

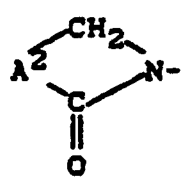

steht und
A¹, A² und R¹ die oben angegebene Bedeutung haben,
erhält, wenn man N-Aryl-Stickstoffheterocyclen der Formel (Id),

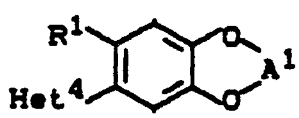

(Id)

in welcher
Het⁴ für einen Rest

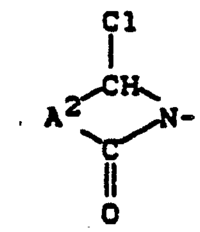

steht und
$A^1$, $A^2$ und $R^1$ die oben angegebene Bedeutung haben,
mit molekularem Wasserstoff in Gegenwart eines Katalysators sowie gegebenenfalls in Gegenwart eines Verdünnungsmittels und gegebenenfalls in Gegenwart eines Säurebindemittels umsetzt.

5. Herbizide und pflanzenwuchsregulierende Mittel, gekennzeichnet durch einen Gehalt an mindestens einem N-Aryl-Stickstoffheterocyclus der Formel (I) gemäß den Ansprüchen 1 und 4.

6. Verfahren zur Bekämpfung von Unkräutern, dadurch gekennzeichnet, daß man N-Aryl-Stickstoffheterocyclen der Formel (I) gemäß den Ansprüchen 1 und 4 auf die Unkräuter und/oder ihren Lebensraum einwirken läßt.

7. Verwendung von N-Aryl-Stickstoffheterocyclen der Formel (I) gemäß den Ansprüchen 1 und 4 zur Bekämpfung von Unkräutern und/oder als Pflanzenwuchsregulatoren.

8. Verfahren zur Herstellung von herbiziden und/oder pflanzenwuchsregulierenden Mitteln, dadurch gekennzeichnet, daß man N-Aryl-Stickstoffheterocyclen der Formel (I) gemäß den Ansprüchen 1 und 4 mit Streckmitteln und/oder oberflächenaktiven Substanzen vermischt.

9. Amine der Formel (IIIa),

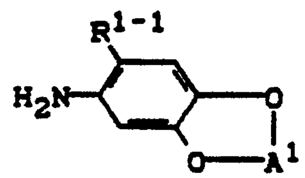

(IIIa)

in welcher
$R^{1-1}$ für Fluor steht und
$A^1$ für einen Rest der Formel

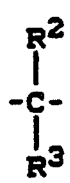 ; 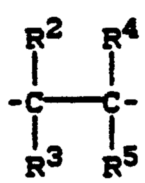 oder 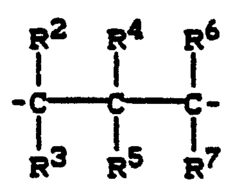 steht,

wobei
$R^2$, $R^3$, $R^4$, $R^5$, $R^6$ und $R^7$ unabhängig voneinander jeweils für Wasserstoff, Halogen, Alkyl oder Halogenalkyl stehen.

10. Nitroverbindungen der Formel (Vb),

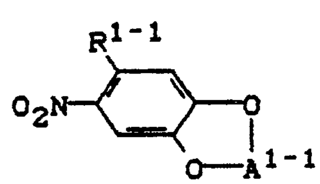

(Vb)

in welcher
$R^{1-1}$ für Fluor steht und

$A^{1-1}$ für einen Rest der Formel

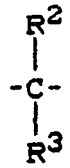 ; 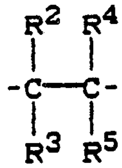 oder 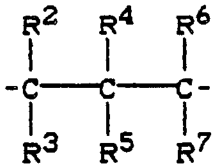 steht, wobei

$R^2$, $R^3$, $R^4$, $R^5$, $R^6$ und $R^7$ unabhängig voneinander jeweils für Wasserstoff, Halogen, Alkyl oder Halogenalkyl stehen und wobei

$A^{1-1}$ jedoch nicht für den Ethandiyl-Rest steht.